(19) Europäisches Patentamt / European Patent Office / Office européen des brevets

(11) **EP 2 406 275 B1**

(12) **FASCICULE DE BREVET EUROPEEN**

(45) Date de publication et mention de la délivrance du brevet:
**29.11.2017 Bulletin 2017/48**

(21) Numéro de dépôt: **10715940.2**

(22) Date de dépôt: **15.03.2010**

(51) Int Cl.:
*C07K 7/06* *(2006.01)*    *A61K 38/08* *(2006.01)*

(86) Numéro de dépôt international:
**PCT/FR2010/050458**

(87) Numéro de publication internationale:
**WO 2010/103254 (16.09.2010 Gazette 2010/37)**

(54) **PEPTIDE PROMOTEUR DE L'ADHESION ET DE LA MIGRATION CELLULAIRE**

PEPTID WELCHES DIE ADHESION UND DIE ZELLULÄRE MIGRATION FÖRDERT

PEPTIDE PROMOTING ADHESION AND CELLULAR MIGRATION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **13.03.2009 FR 0901168**

(43) Date de publication de la demande:
**18.01.2012 Bulletin 2012/03**

(73) Titulaires:
- **Symatese**
  **69630 Chaponost (FR)**
- **Centre National de la Recherche Scientifique (C.N.R.S.)**
  **75794 Paris Cedex 16 (FR)**
- **Université Claude Bernard Lyon I**
  **69622 Villieurbanne Cedex (FR)**

(72) Inventeur: **ROUSSELLE, Patricia**
**F-69005 Lyon (FR)**

(74) Mandataire: **Cabinet Laurent & Charras**
**Le Contemporain**
**50 Chemin de la Bruyère**
**69574 Dardilly Cedex (FR)**

(56) Documents cités:
**WO-A1-2006/025646**

- **VIVES R R ET AL: "A novel strategy for defining critical amino acid residues involved in protein/glycosaminoglycan interactions" JOURNAL OF BIOLOGICAL CHEMISTRY, AMERICAN SOCIETY OF BIOLOCHEMICAL BIOLOGISTS, BIRMINGHAM, US, vol. 279, no. 52, 1 décembre 2004 (2004-12-01), pages 54327-54333, XP009119216 ISSN: 0021-9258 [extrait le 2004-10-12] cité dans la demande**
- **BACHY SOPHIE ET AL: "Syndecan-1 interaction with the LG4/5 domain in laminin-332 is essential for keratinocyte migration" JOURNAL OF CELLULAR PHYSIOLOGY, vol. 214, no. 1, janvier 2008 (2008-01), pages 238-249, XP002528545 ISSN: 0021-9541 cité dans la demande**
- **OKAMOTO OSAMU ET AL: "Normal human keratinocytes bind to the alpha3LG4/5 domain of unprocessed laminin-5 through the receptor syndecan-1" JOURNAL OF BIOLOGICAL CHEMISTRY, AMERICAN SOCIETY OF BIOLOCHEMICAL BIOLOGISTS, BIRMINGHAM, US, vol. 278, no. 45, 7 novembre 2003 (2003-11-07), pages 44168-44177, XP002528550 ISSN: 0021-9258 [extrait le 2003-08-28] cité dans la demande**
- **HASHIMOTO, TADASHI ET AL: "Development of alginate wound dressings linked with hybrid peptides derived from laminin and elastin" BIOMATERIALS , VOLUME DATE 2004, 25(7-8), 1407-1414 CODEN: BIMADU; ISSN: 0142-9612, 2003, XP002549590 cité dans la demande**
- **SUZUKI NOBUHARU ET AL: "Functional sites in the laminin alpha chains" CONNECTIVE TISSUE RESEARCH, vol. 46, no. 3, 2005, pages 142-152, XP9136523 ISSN: 0300-8207**

SEQUENCE LISTING

<110> SYMATESE
CENTRE NATIONAL DE LA RECHERCHE SCIENTIFIQUE
UNIVERSITE CLAUDE BERNARD LYON I

<120> NOUVEAU PEPTIDE PROMOTEUR DE L'ADHESION CELLULAIRE

<130> S283-B-25332 PCT

<150> FR 09/01168
<151> 2009-03-13

<160> 32

<170> PatentIn version 3.3

<210> 1
<211> 10
<212> PRT
<213> artificial sequence

<220>
<223> T+

<400> 1

```
        Lys Lys Leu Arg Ile Lys Ser Lys Glu Lys
        1               5                   10
```

<210> 2
<211> 10
<212> PRT

<213> Artificial sequence

<220>

<221> VARIANT

<222> (2) (6) (8) (10)

<223> may be substituted with others, independenly positive residues, with possibly one being a neutral residue

<220>

<221> variation

<222> (3)..(3)

<223> may be substituted with others, independenly neutral

<220>

<221> variation

<222> (5)..(5)

<223> may be substituted with others, independenly neutral

<220>

<221> variation

<222> (7)..(7)

<223> may be substituted with others, independenly neutral, but not alanine

<220>

<221> variation

<222> (9)..(9)

<223> may be substituted with others, independenly neutral or negative

<220>
<223> A2

<400> 2

```
Lys Ala Leu Arg Ile Lys Ser Lys Glu Lys
1               5                   10
```

<210> 3
<211> 10
<212> PRT
<213> Artificial sequence

<220>
<223> A3

<400> 3

Lys Lys Ala Arg Ile Lys Ser Lys Glu Lys
1               5                   10

<210> 4
<211> 10
<212> PRT
<213> Artificial sequence

<220>
<223> A5

<400> 4

Lys Lys Leu Arg Ala Lys Ser Lys Glu Lys
1               5                   10

<210> 5
<211> 10
<212> PRT
<213> A6

<400> 5

Lys Lys Leu Arg Ile Ala Ser Lys Glu Lys
1               5                   10

<210> 6
<211> 10
<212> PRT
<213> Artificial sequence

<220>
<223> A8

<400> 6

Lys Lys Leu Arg Ile Lys Ser Ala Glu Lys
1               5                   10

<210> 7
<211> 10
<212> PRT
<213> Artificial sequence

<220>
<223> A9

<400> 7

Lys Lys Leu Arg Ile Lys Ser Lys Ala Lys
1               5                   10

<210> 8
<211> 10
<212> PRT
<213> Artificial sequence

<220>
<223> A10

<400> 8

```
Lys Lys Leu Arg Ile Lys Ser Lys Glu Ala
1               5               10
```

<210> 9
<211> 10
<212> PRT
<213> Artificial sequence

<220>
<223> M2

<400> 9

```
Lys Lys Leu Arg Leu Arg Ser Lys Glu Arg
1               5               10
```

<210> 10
<211> 10
<212> PRT
<213> Artificial sequence

<220>
<223> M4

<400> 10

```
Lys Lys Leu Arg His Lys Ser Lys Glu Lys
1               5               10
```

<210> 11
<211> 10
<212> PRT
<213> Artificial sequence

<220>
<223> M5

<400> 11

```
Lys Lys Leu Arg Thr Lys Ser Lys Glu Lys
1               5               10
```

<210> 12
<211> 10
<212> PRT
<213> Artificial sequence

<220>
<223> M6

<400> 12

```
                              Lys Lys Leu Arg Lys Lys Ser Lys Glu Lys
                              1               5                   10
```

<210> 13
<211> 10
<212> PRT
<213> Artificial sequence

<220>
<223> M7

<400> 13

```
                              Lys Lys Leu Arg Ser Lys Ser Lys Glu Lys
                              1               5                   10
```

<210> 14
<211> 10
<212> PRT
<213> Artificial sequence

<220>
<223> M8

<400> 14

```
                              Lys Lys Leu Arg Ile Lys Leu Lys Glu Lys
                              1               5                   10
```

<210> 15
<211> 10
<212> PRT
<213> Artificial sequence

<220>
<223> M9

<400> 15

```
                              Lys Lys Leu Arg Ile Lys Gln Lys Glu Lys
                              1               5                   10
```

<210> 16
<211> 10
<212> PRT
<213> Artificial sequence

<220>
<223> U1

<400> 16

```
                              Lys Lys Leu Arg Ile Gln Ser Lys Glu Lys
                              1               5                   10
```

<210> 17
<211> 10

<212> PRT
<213> Artificial sequence

<220>
<223> U2

<400> 17

```
Lys Lys Leu Arg Ile Lys Ser Gln Glu Lys
1               5                   10
```

<210> 18
<211> 10
<212> PRT
<213> Artificial sequence

<220>
<223> U3

<400> 18

```
Lys Lys Leu Arg Ile Lys Ser Lys Glu Gln
1               5                   10
```

<210> 19
<211> 9
<212> PRT
<213> Artificial sequence

<220>
<223> heparine 1

<400> 19

```
Pro Ser Gly Lys Pro Lys Ser Leu Pro
1               5
```

<210> 20
<211> 9
<212> PRT
<213> Artificial sequence

<220>
<223> heparine 2

<400> 20

```
Thr Ser Val Thr Pro Lys Gln Ser Leu
1               5
```

<210> 21
<211> 12
<212> PRT
<213> Artificial sequence

<220>
<223> syndecan 2/4

<400> 21

```
Asn Ser Phe Met Ala Leu Tyr Leu Ser Lys Gly Arg
1               5               10
```

<210> 22
<211> 10
<212> PRT
<213> Artificial sequence

<220>
<223> A1

<400> 22

```
Ala Lys Leu Arg Ile Lys Ser Lys Glu Lys
1               5               10
```

<210> 23
<211> 10
<212> PRT
<213> Artificial sequence

<220>
<223> A4

<400> 23

```
Lys Lys Leu Ala Ile Lys Ser Lys Glu Lys
1               5               10
```

<210> 24
<211> 10
<212> PRT
<213> Artificial sequence

<220>
<223> A7

<400> 24

```
Lys Lys Leu Arg Ile Lys Ala Lys Glu Lys
1               5               10
```

<210> 25
<211> 10
<212> PRT
<213> Artificial sequence

<220>
<223> U4

<400> 25

```
Lys Lys Leu Arg Ile Gln Ser Gln Glu Lys
1               5               10
```

<210> 26

<211> 10
<212> PRT
<213> Artificial sequence

<220>
<223> U5

<400> 26

```
Lys Lys Leu Arg Ile Lys Ser Gln Glu Gln
1               5                   10
```

<210> 27
<211> 10
<212> PRT
<213> Artificial sequence

<220>
<223> U6

<400> 27

```
Lys Lys Leu Arg Ile Gln Ser Lys Glu Gln
1               5                   10
```

<210> 28
<211> 10
<212> PRT
<213> Artificial sequence

<220>
<223> U7

<400> 28

```
Lys Lys Leu Arg Ile Gln Ser Gln Glu Gln
1               5                   10
```

<210> 29
<211> 10
<212> PRT
<213> Artificial sequence

<220>
<223> U8

<400> 29

```
Gln Gln Leu Arg Ile Lys Ser Lys Glu Lys
1               5                   10
```

<210> 30
<211> 7
<212> PRT
<213> Artificial sequence

<220>
<223> U9

<400> 30

Arg Ile Lys Ser Lys Glu Lys
1               5

<210> 31
<211> 10
<212> PRT
<213> Artificial sequence

<220>
<223> M1

<400> 31

Lys Lys Leu Lys Ile Arg Ser Gln Glu Lys
1               5               10

<210> 32
<211> 10
<212> PRT
<213> Artificial sequence

<220>
<223> M3

<400> 32

Phe Lys Lys Arg Thr Lys Ser Lys Glu Asp
1               5               10

**Description**

Il est décrit un nouveau peptide de séquence définie présente sur la chaîne alpha3 du globule LG4 de la laminine 332 (laminine 5), ainsi que des peptides de séquence homologue audit peptide de séquence définie, et capables de se lier au récepteur syndécan-1. Elle a également pour objet une composition pharmaceutique ou cosmétique contenant ledit peptide. Elle concerne enfin l'utilisation du peptide dans le domaine de la cicatrisation et régénération tissulaires ou comme adjuvant dans des milieux de culture destinés à la reconstruction épidermique ou épithéliale.

Chaque année des dizaines de millions de patients souffrent de pertes tissulaires ou de déficiences d'organes. Bien que la transplantation des organes et des tissus concernés permette de répondre à une partie de la demande, cette approche thérapeutique est extrêmement limitée par le manque de donneur et par le rejet potentiel des allogreffes nécessitant des traitements immunosuppresseurs à long terme.

Depuis une dizaine d'années, l'intérêt pour le génie tissulaire a connu un essor incomparable tant sur le plan de la recherche fondamentale que sur celui de la définition et de la mise au point de "tissus artificiels" qui commencent à être proposés pour des applications cliniques par diverses sociétés. Le génie tissulaire est un domaine pluridisciplinaire qui applique les principes de l'Ingénierie et des Sciences de la Vie pour le développement de substituts biologiques en vue de restaurer, maintenir ou améliorer une fonction tissulaire.

Le génie tissulaire associe des biomatériaux naturels ou synthétiques biodégradables, des cellules non différenciées (cellules souches) ou différenciées (kératinocyte, fibroblaste, chondrocyte ...) et (ou) des molécules actives comme des facteurs de croissance ou des molécules d'adhérence. En effet, l'adhérence cellulaire est une étape indispensable et déterminante au succès de l'implantation des biomatériaux dans l'organisme humain. Si celle-ci a pu être améliorée par la mise au point de surfaces hautement interactives, il n'en reste pas moins qu'elle reste dépendante de la présence, ou de la synthèse par les cellules, de protéines matricielles d'adhérence.

Par ailleurs, 5 millions de personnes ont besoin annuellement de pansements cicatrisants pour le traitement des plaies difficiles (escarres et ulcères de jambes). De nombreux pansements supposés favoriser la cicatrisation cutanée sont actuellement sur le marché et les recherches dans ce domaine se multiplient. Malgré les efforts fournis dans le traitement et la prévention de l'infection et de la déshydratation, de nombreux problèmes subsistent dans la prise en charge des problèmes de cicatrisations compliquées. En effet, le problème de la cicatrisation des plaies chroniques va en s'accroissant du fait de l'allongement de la durée de la vie et du vieillissement des populations. Une nouvelle ère s'annonce avec la mise au point de pansements porteurs de molécules d'adhérence.

La cicatrisation cutanée est un phénomène complexe au cours duquel des événements dermiques et épidermiques sont étroitement impliqués et n'est pas, comme on l'a longtemps cru, un simple processus linéaire au cours duquel des facteurs de croissance sont synthétisés pour activer la prolifération et la migration cellulaires. C'est au contraire la résultante de plusieurs processus dynamiques et interactifs mettant en jeu des facteurs solubles, des éléments sanguins, des composants de la matrice extracellulaire (MEC) et des cellules parenchymateuses et épithéliales (Clark, 1996).

Les interactions entre les cellules et la MEC jouent un rôle dans le contrôle du comportement cellulaire en contrôlant l'expression de certains gènes, ou en modulant la migration, la prolifération, la différenciation ou la mort cellulaire programmée (Hynes, 1992). Ce contrôle du comportement cellulaire est crucial au cours des remaniements tissulaires. Lorsqu'il y a une blessure cutanée, la jonction dermo-épidermique (JDE) est endommagée et ses molécules constitutives sont détruites ou dégradées par des enzymes spécifiques. Le derme joue un rôle fondamental au cours de la ré-épithélialisation en produisant des facteurs solubles destinés aux cellules épithéliales et des molécules matricielles informatives avec lesquelles les kératinocytes ont un contact direct. Dans des conditions favorables, la ré-épithélialisation épidermique débute quelques heures après le traumatisme. La réaction inflammatoire entraîne l'apparition sur le lit de la plaie de molécules matricielles et les kératinocytes des structures épithéliales résiduelles migrent rapidement sur le lit de la plaie.

Au cours du processus de réparation, les kératinocytes migrants perdent leur polarité apicale-basale, adhèrent à la matrice provisoire, et émettent des pseudopodes qui leur permettent d'effectuer une migration latérale (Larjava *et al.,* 1993). Lorsque l'épithélium à recouvert le lit de la plaie, les protéines de la JDE réapparaissent de façon séquentielle et ordonnée à partir des marges de la plaie jusqu'au centre. Les kératinocytes basaux ré-adoptent alors un phénotype stationnaire à polarité apicale, fermement ancrés à la JDE par l'intermédiaire des hémidesmosomes (HDs) reconstitués sur leur surface basale. Ce contrôle de la migration et de l'adhésion des kératinocytes basaux s'effectue par des interactions spécifiques entre certains motifs peptidiques présents sur les molécules matricielles et des récepteurs transmembranaires, regroupés dans des complexes d'adhérence et dont le domaine intracellulaire est relié aux constituants du cytosquelette (Aumailley *et al.,* 1996). Les complexes d'adhérence cellulaires sont de larges agrégats multimoléculaires permettant le transfert bidirectionnel de l'information au travers de la membrane cellulaire. Ces complexes, constitués de protéines extracellulaires, transmembranaires (intégrines et syndécans), cytosoliques et cytosquelettiques, sont des structures hautement dynamiques et stabilisées de façon plus ou moins transitoire par des interactions spécifiques entre les divers constituants. L'assemblage et le désassemblage des complexes d'adhérence sont finement régulés pour permettre à la cellule d'effectuer des opérations primordiales comme l'adhérence, la migration, la prolifération et la différenciation.

Dans le large éventail de ligands extracellulaires, la laminine 332 (LN332) également désignée laminine 5 joue un rôle fondamental au cours de ces deux processus (Aumailley and Rousselle, 1999 ; Ghohestani *et al.,* 2001). Cette molécule d'adhérence a une double fonction : selon une régulation finement contrôlée, elle est capable d'induire une adhérence cellulaire forte et statique ou au contraire une adhérence de faible affinité, transitoire permettant la migration cellulaire. Cette propriété est très bien illustrée dans la peau puisque la LN332 est d'une part, responsable de l'ancrage de l'épiderme, et d'autre part impliquée dans la migration des kératinocytes lors des processus de cicatrisation.

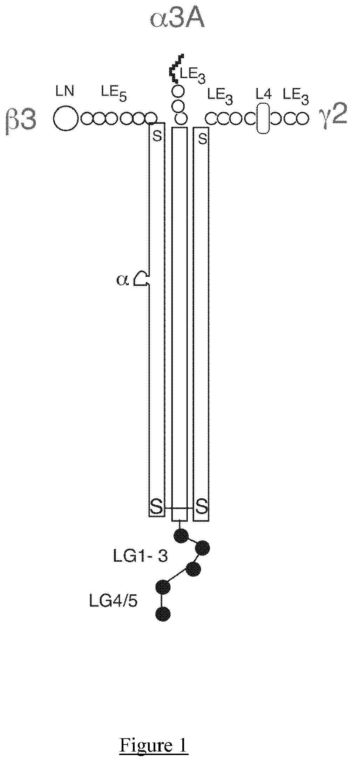

La LN332, constituée par l'assemblage de 3 sous-unités alpha3, beta3 et gamma2 (figure 1). Elle est la protéine d'adhérence majeure et irremplaçable de l'épiderme où elle a été identifiée comme étant le composant majeur des filaments d'ancrage, structures qui relient les HDs à la partie basale de la JDE dans la peau (Rousselle *et al.*, 1991). Elle intervient dans l'adhérence d'un grand nombre d'autres cellules épithéliales de l'organisme (Rousselle et Aumailley, 1994). Elle est synthétisée exclusivement par les cellules épithéliales sous la forme d'un précurseur de 460 kDa (pre-LN332). La chaîne alpha3 plus longue que les 2 autres comporte à son extrémité carboxy-terminale un domaine globulaire constitué de 5 répétitions de résidus basiques apparaissant comme 5 globules appelés LG1 à LG5 (LG1-2-3-4-5). Ces domaines globulaires sont très importants puisqu'ils comportent les domaines responsables de l'adhérence des kératinocytes.

Le document WO2000/66731 décrit la production de laminine 5 entière sous forme recombinante.

Le document WO2006/018551 décrit quant à lui l'implication de la séquence TALRIRATYGEY présente sur la chaine gamma2 de l'extrémité N terminale de la laminine 5 dans l'adhérence des kératinocytes de l'épiderme.

Le document WO2006/025646 décrit une protéine recombinante rLG3-His6 de 311 aa, comprenant le domaine globulaire nommé LG3, apte à promouvoir une adhésion et une migration cellulaire.

Dans la présente demande, le Demandeur s'est intéressé à l'extrémité carboxy-terminale de la chaîne alpha3 de la pre-LN332.

Dans les conditions physiologiques, chacune des sous-unités alpha3 et gamma2 de la LN332 subit un clivage post-traductionnel extracellulaire de leur extrémité carboxy-terminale et amino-terminale respectivement (Marinkovich *et al.,* 1994 ; Goldfinger et al., 1998 ; Amano *et al.,* 2000). Ces différents clivages aboutissent à la forme mature qui joue un rôle crucial dans la cohésion épithélio-mésenchymateuse tant sur le plan de l'organisation supra-moléculaire au niveau des extrémités amino-terminales que des interactions cellulaires portées par les domaines LG (Aumailley et Rousselle, 1999). En effet, la maturation de la chaine alpha3, qui résulte de la coupure du tandem LG4/5, induit des changements fonctionnels déterminants de la LN332 puisqu'elle permet l'adhérence des cellules épithéliales adjacentes par l'intermédiaire des intégrines alpha6béta4 et induit l'assemblage des structures d'adhérence stables que sont les hémidesmosomes. (Sonnenberg *et al.*, 1993 ; Rousselle et Aumailley, 1994; Niessen *et al.,* 1994). La LN332 mature constituerait ainsi, une barrière à la motilité cellulaire (Baker *et al.,* 1996 ; Goldfinger *et al.,* 1998 ; Ryan *et al.,* 1999). La pre-LN332 est quant à elle capable, par le biais des globules LG4/5 de la chaine alpha3 précurseur, d'empêcher la formation des hémidesmosomes et d'induire la migration cellulaire (Ryan *et al.,* 1999 ; Goldfinger *et al.,* 1999 ; Bachy *et al.,* 2008). Des études *in vivo* de la cicatrisation cutanée ont montré une expression accrue des différentes chaînes de la LN332 par les kératinocytes localisés dans la zone de colonisation de la plaie (Ryan *et al.*, 1994), celle-ci étant présente dans la matrice extracellulaire sous sa forme précurseur (Ryan et al., 1994; Lampe et al., 1998; Nguyen et al., 2000). La pre-LN332 est ainsi présente dans la matrice provisoire mais elle est totalement absente dans les lames basales organisées où elle apparaît sous sa forme maturée (Lampe et al., 1998; Goldfinger et al., 1999; Tungall et al., 2002). Par ailleurs, si les études *in vitro* ont très tôt rapporté une sur-expression et une synthèse accrue de la LN332 par des kératinocytes mobiles (Rousselle et al., 1991), son implication par le biais de la forme précurseur a été plus tardive (Nguyen et al., 2000 ; Décline and Rousselle, 2001). L'ensemble de ces données montre que le domaine LG4/5 influence la migration cellulaire et joue un rôle dans le processus de réparation épithéliale. Des travaux dont ceux des inventeurs de la présente demande, ont montré que la pre-LN332 coopère avec l'intégrine alpha3béta1 pour contrôler la polarisation et la migration cellulaires (Goldfinger *et al.,* 1998; Décline and Rousselle, 2001 ; Frank and Carter, 2004 ; Bachy *et al.,* 2008). Par ailleurs, une étude propose un rôle du fragment LG4/5 dans le dépôt et la rétention de la pre-LN332 dans la matrice extracellulaire (Sigle *et al.,* 2004).

Enfin, il a été montré que le domaine LG4/5 de la pre-LN332 interagit avec un récepteur de la famille des protéoglycannes, le syndécan-1, au cours de la migration des kératinocytes (Okamoto *et al.,* 2003 ; Bachy *et al.,* 2008).

Dans l'épiderme, le syndécan-1 est localisé dans la région péricellulaire des kératinocytes des couches suprabasales et est assez peu exprimé dans la couche basale. De façon remarquable, l'expression du syndécan-1 est fortement activée au cours de la cicatrisation et est principalement localisée au niveau des bords des plaies (Elenius *et al.,* 1991; Oksala *et al.*, 1995; Jaakkola *et al.,* 1998). Ce patron d'expression est spécifique du syndécan-1 puisqu'il n'a pas été retrouvé pour les autres syndécans (Gallo et al., 1996). Par ailleurs, les souris déficientes pour le gène codant le syndécan-1 présentent des défauts de prolifération et de migration des kératinocytes pendant la cicatrisation (Stepp *et al,* 2002). Les syndécans sont connus pour intervenir dans des processus cellulaires comme la migration et ils sont connus comme étant des co-récepteurs, coopérant avec des intégrines et des facteurs de croissance (Bernfield *et al.,* 1999 ; Woods *et al.,* 2000). L'ensemble de ces éléments conforte l'hypothèse selon laquelle le domaine LG4/5 de la pre-LN332 représenterait un ligand préférentiel du syndécan-1 au cours de la cicatrisation et serait à la base d'une cascade de signalisation intracellulaire favorable au processus de réparation épithéliale.

Le site de clivage du fragment LG4/5 sur la chaine alpha3 a été déterminé (Décline *et al.*, 2003) et le fragment LG4/5 a été produit sous forme recombinante (Okamoto *et al.*, 2003). Des expériences d'adhérence et de migration cellulaires menées avec le fragment LG4/5 ont permis de montrer que ce domaine est impliqué dans la migration cellulaire et qu'un récepteur membranaire, le syndécan-1, est spécifiquement impliqué dans sa reconnaissance (Okamoto *et al.,* 2003, Bachy *et al.*, 2008). Les syndécans appartiennent à une famille de récepteurs membranaires à chaînes d'héparane sulfate exprimée à la surface de toutes les cellules adhérentes. Ces récepteurs ont été décrits comme étant des "facilitateurs" moléculaires capables de moduler, par une signalisation intracellulaire reliée au cytosquelette, l'activité des intégrines. L'adhésion cellulaire au fragment LG4/5 est dépendante des chaînes d'héparane sulfate et de chondroitine sulfate présentes sur le syndécan-1. Si ces deux chaines de glycosaminoglycannes se lient au fragment LG4/5 avec des affinités différentes, elles reconnaissent le même domaine d'interaction dans le globule LG4/5 (Okamoto *et al.,* 2003). La pre-LN332 induit la migration des kératinocytes humains normaux par le biais de l'interaction du syndécan-1 avec le domaine LG4/5. L'adhérence syndécan-1 dépendante au domaine LG4/5 de la pre-LN332, induit une réorganisation du cytosquelette des keratinocytes humain normaux conduisant à la formation de filopodes et de microspicules, prolongements cytoplasmiques protrusifs caractéristiques de la migration cellulaire. Cette étape précoce est accompagnée d'une activation des GTPases Rac et Cdc42, GTPases connues pour être impliquées dans la formation de complexes d'adhérence transitoires spécifiques de la migration cellulaire (Bachy *et al.,* 2008).

L'obtention de la structure du module LG5 de la chaîne alpha2 de la LN211 (Tisi *et al*., 2000 ; Timpl *et al.,* 2000), à permis d'établir un modèle structural de base de ces modules LG. Ces modules sont organisés sous la forme de 14 feuillets béta A à N reliés les un aux autres par des boucles orientées à l'extérieur de la structure. A l'aide d'une technologie basée sur le couplage covalent du fragment LG4/5 recombinant sur des billes recouvertes d'héparine suivi d'une fragmentation enzymatique de la protéine, trois séquences correspondant à des « zones de liaison à l'héparine » ont été identifiés (Vives *et al*., 2004), respectivement les séquences KKLRIKSKEK, PSGKPKSLP et TSVTPKQSL. Dans ce document, les expérimentations ont été conduites à partir de la protéine native et les sites de liaison ont été identifiés sans pour autant qu'ils aient été isolés à l'état de peptide.

Parallèlement les documents JP2006063033 et Utani et al, 2001, décrivent la séquence NSFMALYLSKGR (du résidu 1412 au résidu 1423 de la séquence de la chaine alpha 3) localisée dans le module LG4, comme inductrice de l'adhérence cellulaire via les syndécans 2 et 4.

Le Demandeur a démontré que la séquence KKLRIKSKEK (SEQ ID 1) (du résidu 1433 au résidu 1442 de la séquence de la chaîne alpha3 et localisé au niveau d'une boucle reliant les feuillets béta F et G dans le globule LG4 est le site de liaison au syndécan-1 dans le module LG4 puisque (1) elle est la seule capable d'induire l'adhérence cellulaire syndécan-1 dépendante et (2) elle est capable de lier le syndécan-1 de façon spécifique et équivalente au domaine LG4/5 entier.

L'identification d'un peptide fonctionnel de taille aussi réduite était loin d'être évidente, comme le démontre le document Urushibata et al. (Biochemistry, 2099, 48, 10522-10532), dans lequel les auteurs ont analysé l'activité biologique de 107 peptides synthétiques appartenant au domaine G de la chaîne alpha3 de la laminine, sans mettre en évidence celui faisant l'objet de la présente invention.

Dès lors, l'invention a tout d'abord pour objet un peptide synthétique ou analogue tel que défini ci-après.

Le document US 2005/0059602 décrit effectivement la séquence A3G78 constituée de 12 aa et comprenant la séquence SEQ ID 1. Plus précisément, les peptides suivants sont donc exclus :

- KKLRIKSKEK ;
- DGKKLRIKSKEK.

Le document Hashimoto et al. (Biomaterials 25 (2004), 1407-1414) décrit des peptides hybrides liés à des pansements en alginate. Certains présentent une taille de 10 aa, avec un résidu arginine (R) en position 4. Toutefois, ces peptides possèdent une acétyl-lysine (ac-K) en position 1 et ne sont pas susceptibles de se lier au récepteur syndécan-1. Ne sont donc également pas visés par la présente invention les peptides suivants :

- Ac-KSIRVAVAPG
- Ac-KSIRIAIAPG
- Ac-KSIRVGVGPG
- Ac-KSIRIGIGPG.

La liaison au syndécan-1 peut être facilement révélée par toute technique connue de l'homme du métier, en particulier par technique de chromatographie d'affinité. Un test de ce type peut par exemple consister à fixer le peptide à tester sur des billes, puis les incuber avec des lysats de kératinocytes. Les protéines cellulaires ainsi fixées sur le peptide peuvent ensuite être analysées par électrophorèse SDS PAGE suivie d'une immunodétection pour évaluer le contenu en syndécan-1.

Il est démontré dans la suite de la description par le biais d'une analyse quantitative que l'adhérence cellulaire révélée par le peptide KKLRIKSKEK (SEQ ID 1) est plus efficace que celle révélée par le peptide NSFMALYLSKGR puisque d'une part, l'adhérence cellulaire est induite par une quantité plus basse de peptide immobilisée et d'autre part, le nombre total de cellules adhérées est supérieur de 200% sur le peptide KKLRIKSKEK quelle que soit la quantité de peptide immobilisé.

Dans la suite de la description et dans les revendications, par « peptide analogue », on désigne le peptide de l'invention sous forme modifiée à condition que ledit peptide garde les caractéristiques énoncées ci-dessous. Les modifications peuvent être d'ordre conformationnel. Le peptide peut ainsi se présenter sous forme oligomérisée, repliée, cyclisée. Les modifications peuvent être également d'ordre chimique. Ainsi par exemple, un peptide sur lequel serait greffé un motif d'intérêt ou dont la chaine latérale serait modifié chimiquement est également couvert par la présente invention.

Ainsi, l'invention concerne un peptide comprenant ou constitué de la séquence $Kaa_2aa_3Raa_5aa_6aa_7aa_8aa_9aa_{10}$ dans laquelle, à pH physiologique :

- $aa_2$, $aa_6$, $aa_8$, $aa_{10}$ sont des résidus de charge positive ; au plus un d'entre eux pouvant être substitué par un résidu de charge neutre,
- $aa_3$ est un résidu de charge neutre,
- $aa_5$ est un résidu de charge neutre,
- $aa_7$ est un résidu de charge neutre à l'exception de l'alanine, et
- $aa_9$ est un résidu de charge négative ou un résidu de charge neutre

la séquence étant apte à se lier au récepteur syndécan-1,
à l'exception des peptides comprenant ou constitué de la séquence KKLRIKSKEK (SEQ ID 1).

Dans la suite de la description et dans les revendications, par résidu de charge neutre on désigne les acides aminés suivants : phénylalanine, méthionine, tryptophane, valine, leucine, isoleucine, alanine, proline, glycine, cystéine, asparagine, glutamine, sérine, thréonine, tyrosine.

De même, par résidu de charge positive, on désigne les acides aminés suivants : lysine, arginine et histidine.

Enfin, par résidu de charge négative, on désigne les acides aminés suivants : acide aspartique, acide glutamique.

Le Demandeur a obtenu de très bons résultats d'adhésion cellulaire avec les peptides avantageusement visés par l'invention, contenant ou étant constitués par les séquences suivantes :

- A2 : KALRIKSKEK (SEQ ID 2)
- A3 : KKARIKSKEK (SEQ ID 3)
- A5 : KKLRAKSKEK (SEQ ID 4)
- A6 : KKLRIASKEK (SEQ ID 5)
- A8 : KKLRIKSAEK (SEQ ID 6)
- A9 : KKLRIKSKAK (SEQ ID 7)
- A10 : KKLRIKSKEA (SEQ ID 8)
- M2 : KKLRLRSKER (SEQ ID 9)
- M4 :KKLRHKSKEK (SEQ ID 10)
- M5 : KKLRTKSKEK (SEQ ID 11)
- M6 : KKLRKKSKEK (SEQ ID 12)
- M7 : KKLRSKSKEK (SEQ ID 13)
- M8 : KKLRIKLKEK (SEQ ID 14)
- M9 : KKLRIKQKEK (SEQ ID 15)
- U1 : KKLRIQSKEK (SEQ ID 16)
- U2 : KKLRIKSQEK (SEQ ID 17)
- U3 : KKLRIKSKEQ (SEQ ID 18).

L'invention a également pour objet un peptide synthétique

- contenant au plus 30 acides aminés et comprenant ou constitué de la séquence KKLRIKSKEK (SEQ ID 1), ou
- comprenant ou constitué de la séquence $Kaa_2aa_3Raa_5aa_6aa_7aa_8aa_9aa_{10}$, dans laquelle, à pH physiologique :

- aa$_2$, aa$_6$, aa$_8$, aa$_{10}$ sont des résidus de charge positive ; au plus un d'entre eux pouvant être substitué par un résidu de charge neutre,
- aa$_3$ est un résidu de charge neutre,
- aa$_5$ est un résidu de charge neutre,
- aa$_7$ est un résidu de charge neutre à l'exception de l'alanine, et
- aa$_9$ est un résidu de charge négative ou un résidu de charge neutre,

la séquence étant apte à se lier au récepteur syndécan-1, à l'exception des peptides comprenant ou constitué de la séquence KKLRIKSKEK (SEQ ID 1) pour utilisation comme médicament. Selon un mode de réalisation particulier, un tel peptide contient ou est constitué d'une séquence choisie dans le groupe constitué des séquences SEQ ID 2 à SEQ ID 18.

Un tel peptide est avantageusement utilisé dans la cicatrisation de la peau, en suite notamment de pathologie de la peau ou de traumatisme.

Les pathologies cutanées concernées sont notamment :

➢ Ulcères, escarres,

➢ Traitement de la peau après des procédures dermatologiques, par exemple à but esthétique pour le traitement du vieillissement, du melasma, traitement laser $CO_2$, ou Erbium, laser fractionnel, peeling chimique, microdermabrasion

➢ Pathologie de la jonction dermo-épidermique, par exemple épidermolyse bulleuse,

➢ Psoriasis.

Les traumatismes concernés sont notamment :

➢ Brûlure du deuxième degré, superficiel à profond, du troisième degré dermabrasion, ampoules

➢ Reconstruction dermique, chirurgie plastique, greffe de peau

➢ Zones donneuses de greffe de peaux

Le peptide pour utilisation comme médicament, tel que défini ci-dessus, peut également servir à la régénération tissulaire, notamment pour le repulpage ou le réépaississement de la peau. Dans ce contexte, il a par exemple un intérêt dans le comblement de rides.

L'invention a également pour objet une composition pharmaceutique ou cosmétique comprenant le peptide précédemment décrit en relation avec son utilisation comme médicament, susceptible d'adopter plusieurs formes.

Dans une première forme de réalisation, la composition pharmaceutique de l'invention s'apparente davantage à un dispositif médical, en l'espèce un support sur lequel est greffé ou déposé, ou à l'intérieur duquel est incorporé le peptide synthétique de l'invention, le support se présentant sous la forme d'un film ou d'une matrice constitués d'un matériau biologique choisi dans le groupe comprenant collagène, gélatine, polysaccharide, acide hyaluronique, cellulose, carboxyméthylcellulose, pectine, chitosane, derme humain ou animal acellularisé, ou d'un matériau synthétique choisi dans le groupe comprenant silicone, polyuréthane, PLLA, ou d'un matériau textile du type pansement choisi dans le groupe comprenant coton, polyester, polyamide.

De tels dispositifs sont notamment utilisés pour la reconstruction de la peau dans des brûlures du troisième degré. Ainsi, il est décrit un procédé de reconstruction de la peau, en particulier dans des brûlures du troisième degré, consistant à appliquer le dispositif médical ci-avant décrit au contact de la peau.

Dans un second mode de réalisation, la composition pharmaceutique ou cosmétique de l'invention se présente sous la forme d'une crème, d'un hydrogel, d'une solution, d'une formulation injectable ou d'un spray comprenant éventuellement des kératinocytes autologues ou des cellules mésenchymateuses.

Dans le cas de la solution, de la crème ou de l'hydrogel, le ou les peptides sont incorporés dans ces préparations destinées par exemple à être badigeonnée sur la peau lors de greffe de peaux au niveau des sites donneurs et receveurs de greffons épidermiques ou dermoépidermiques mais également pour une cicatrisation de la peau après éraflure, brûlure du deuxième degré, dans le cas des plaies chroniques ou toute procédure médicale de traitement de l'épiderme.

Dans une autre application, le ou les peptides sont incorporés dans une formulation injectable pour le traitement du vieillissement cutané par exemple en mésothérapie. Une application topique peut également être envisagée.

La forme spray est plus particulièrement adaptée dans des applications de génie tissulaire avant la pose de feuillets épidermiques ou peaux totales autologues ou allogéniques, par exemple pour le traitement des grands brûlés ou la cicatrisation des plaies récalcitrantes type ulcères. Dans un mode de réalisation particulier, la forme spray peut également contenir, outre le peptide, des kératinocytes autologues. Le traitement effectué notamment sur les grands brûlés peut être exécuté en un temps ou deux temps.

Traitement en un temps :

◦ prélèvement d'un lambeau d'épiderme suivi de la digestion enzymatique de ce tissu puis récupération d'une suspension cellulaire à laquelle le ou les peptides sont ajoutés. Le mélange cellules/ peptides est alors pulvérisé sur la plaie à traiter.

Traitement en deux temps :

◦ prélèvement d'un lambeau d'épiderme suivi d'une digestion enzymatique du lambeau épidermique. Les kératinocytes sont isolés puis mis en culture pour amplification. Récupération des cellules sous forme de suspension à laquelle le ou les peptides sont ajoutés. Le mélange est ensuite pulvérisé sur la plaie à traiter.

L'invention a également pour objet un milieu de culture contenant le peptide précédemment décrit en relation avec son utilisation comme médicament, destiné à la culture *in vitro* de cellules épithéliales ou mésenchymateuses.

Plus précisément, le ou les peptides peuvent être ajoutés à des milieux de culture spécifiques pour la culture *in vitro* de cellules épithéliales ou mésenchymateuses (kératinocytes et fibroblastes entre autres) soit directement dans le milieu de culture lors de sa fabrication ou sous la forme de complément ajouté extemporanément dans le milieu de culture lors de l'utilisation.

Un tel milieu de culture peut être destiné à la culture de kératinocytes et d'autres cellules épithéliales (épithélium cornéen, épithélium buccal ou vaginal) pour des applications médicales, scientifiques, de recherche ou de pharmacotoxicologie, l'ajout du ou des peptides étant de nature à faciliter et/ou raccourcir les étapes de culture, et/ou améliorer la qualité des cultures et construction cellulaire (tapis cellulaire, pseudoépiderme, épithéliums reconstruits).

Un milieu type de culture de kératinocytes est par exemple décrit dans le brevet US5654135.

Les applications des cultures de keratinocytes ou des cellules épithéliales sont par exemple:

■ Réalisation d'épiderme ou d'épithélium reconstruit pour des tests de molécules pharmacologiques ou cosmétiques ou de préparations pharmacologiques ou cosmétiques (en s'affranchissant de modèles animaux)
■ Pour le traitement des grands brûlés ou le traitement de la cicatrisation (ex ulcères variqueux ou ulcère diabétique), par la réalisation de spray de cellules ou de feuillets épidermiques. Le peptide de l'invention peut également être mis en oeuvre dans un milieu de culture de kératinocytes humains normaux destiné au maintien en survie des substituts cutanés avant greffe, en particulier à un grand brûlé.
■ A des fins de recherches, thérapie génique, étude des kératinocytes, test de nouveaux actifs, facteurs de croissances...
■ Pour la constitution de banques de cellules

Le milieu de culture contenant le ou les peptides peut être destiné à la culture de fibroblastes *in vitro* en vue de la réalisation de peaux totales, et préalablement à l'ensemencement des kératinocytes et ensuite dans les phases de réalisation du pseudoépiderme. Le ou les peptides permettent une meilleure adhésion et étalement des fibroblastes limitant ainsi la perte des fibroblastes immédiatement après ensemencement ainsi qu'une plus grande mobilité des fibroblastes ensemencés.

La durée d'obtention d'un derme équivalent de qualité est réduite et permet d'obtenir une peau totale plus rapidement.

*In vivo* la présence du peptide au sein de la matrice tridimensionnelle permet une meilleure adhésion et étalement des fibroblastes ainsi qu'une limitation de la formation du tissu de granulation lors de la cicatrisation cutanée.

L'invention a également pour objet un support de culture de cellules épithéliales telles que par exemple des kératinocytes contenant le peptide en relation avec son utilisation comme médicament. Les supports peuvent se présenter sous la forme de membranes ou matrices pouvant servir à la réalisation de pseudo épiderme ou de peaux totales pouvant servir aux tests toxicologiques de produits, molécules ou formulations cosmétiques ou pharmaceutiques.

Le ou les peptides permettent de diminuer les pertes à l'ensemencement, augmenter la vitesse de reconstruction de l'épiderme et améliorer la qualité structurelle de l'épiderme reconstruit.

Plus précisément le support de culture peut se présenter sous la forme de boites de cultures en polystyrène sur lesquelles sont adsorbés, greffés les peptides.

Le support de culture peut également se présenter sous la forme d'une membrane ou film de biopolymère utilisé pour la reconstruction d'épiderme équivalent, par exemple sur une membrane de collagène de type I et de collagène de type IV, sur lesquelles sont adsorbés ou greffés les peptides.

Dans les applications où le peptide est greffé en surface d'un film, d'une matrice, d'une membrane, d'une boite de culture cellulaire, il peut être ajouté à la partie C ou N terminale un groupement qui permettra une liaison covalente sur le support. Ce groupement réactif vis-à-vis du support sera éventuellement espacé du peptide par un bras espaceur.

Le groupement pourra consister par exemple en greffage du motif Cys-Gly-Gly sur la partie N terminale pour une réaction sur un chitosane chloroacétylé comme décrit dans Masuda *et al.* (2009).

Il peut aussi être ajouté un bras biotine qui permettra un greffage non covalent sur un support fonctionnalisé avec de l'avidine.

Le support de culture peut également se présenter sous la forme d'une membrane textile ou matrice poreuse sur laquelle sont adsorbés ou greffés les peptides.

Le support de culture peut également se présenter sous la forme de gels, par exemple des gels de collagène acido-soluble sur lesquels sont adsorbés ou greffés les peptides.

Le ou les peptides peuvent aussi être apportés indépendamment du milieu de culture sous la forme d'une solution de coating, par exemple pour le coating de boite de culture ou pour le coating de membrane de biopolymère utilisée pour la reconstruction d'épiderme équivalent, par exemple sur une membrane de collagène de type I et de collagène de type IV.

L'invention et les avantages qui en découlent ressortiront bien des exemples qui suivent à l'appui des figures annexées.

Figure 1 : Présentation de la laminine 332

Les chaînes alpha3, béta3 et gamma2 sont assemblées pour former une hélice alpha super-enroulée au niveau de leur extrémité C-terminale. La chaine alpha 3, plus longue que les deux autres, comporte à son extrémité C-terminale une série de 5 domaines globulaires nommés LG1 à LG5. La molécule est organisée en domaines : LN (laminin N-terminal domain), LE (laminin epidermal growth factor like domain), L4 (laminin domain 4), LG (laminin globular domain), LCC (laminin coiled-coil).

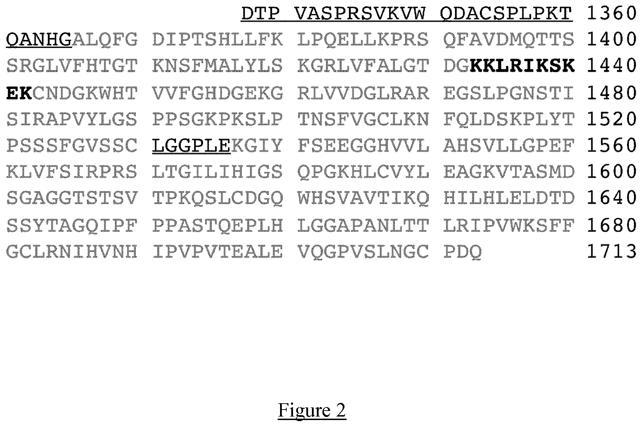

Figure 2 : Séquence peptidique du domaine LG4/5 et localisation du site de liaison au syndécan-1.

Séquence du domaine LG4/5 de la chaine alpha3 de la pre-LN332. La séquence N-terminale commence à partir du site de clivage au niveau de l'acide aspartique en position 1338 et se termine par un acide glutamique en position 1713. Les régions soulignées représentent des domaines linéaires « espaceurs » présents entre les globules. Le domaine de liaison au syndécan-1 KKLRIKSKEK (SEQ ID 1) est représenté en noir foncé, position 1433 à position 1442.

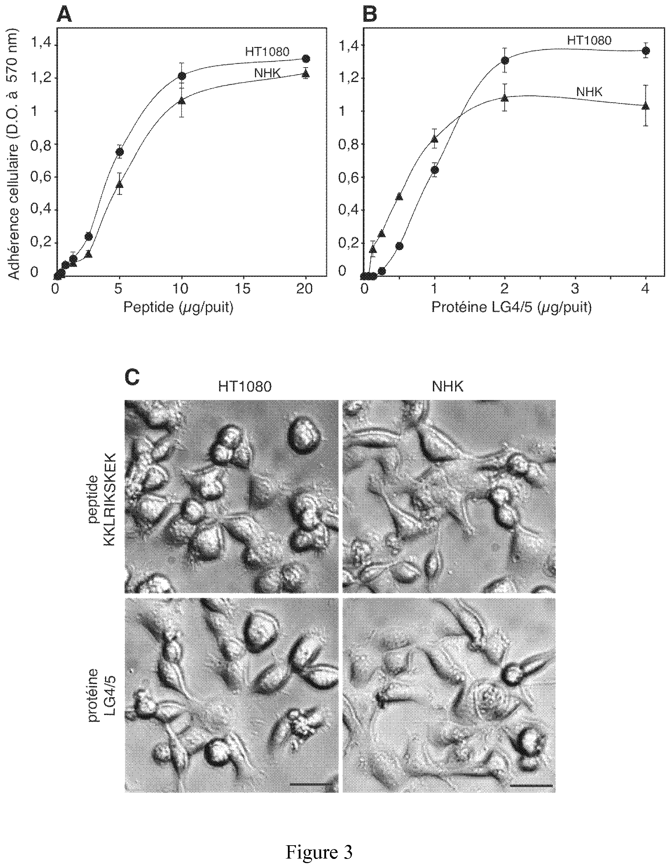

Figure 3 : Adhérence des kératinocytes humains normaux et des cellules HT1080 au fragment LG4/5 et au peptide KKLRIKSKEK (SEQ ID 1).

(A, B) Adhérence cellulaire dose dépendante des kératinocytes humains normaux et des cellules de la lignée HT1080 au peptide KKLRIKSKEK (SEQ ID 1) et à la protéine LG4/5. Le peptide (A) et la protéine LG4/5 (B) ont été immobilisés sur des plaques 96 puits aux concentrations indiquées en abscisse. $6.10^4$ cellules ont été déposées dans chaque puits et les plaques ont été incubées à 37 °C pendant 1 heure. Après lavages, les cellules adhérées ont été fixées et l'adhérence cellulaire a été mesurée comme décrit dans la partie méthodologie. (C) Observation des kératinocytes humains normaux et des cellules HT1080 adhérés au peptide KKLRIKSKEK (SEQ ID 1) et à la protéine LG4/5. L'observation a été faite avec un microscope Axiovert 40 Zeiss équipé d'un bloc interférentiel PlasDic. Barre, 50 μm.

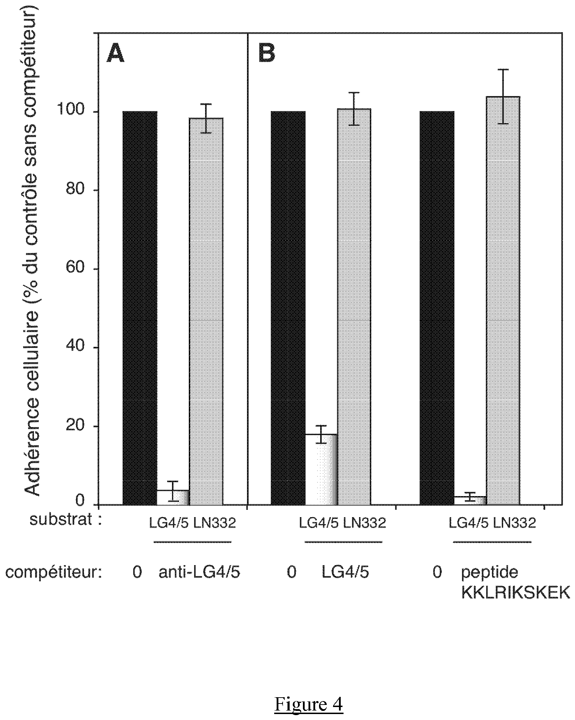

Figure 4 : Effet du peptide KKLRIKSKEK (SEQ ID 1) sur l'adhérence des cellules au fragment LG4/5 et à la laminine 332.

Les protéines LG4/5 (barres blanches) et LN332 (barres grises) (1 μg/puits) ont été adsorbées dans des plaques 96 puits. (A) Effet de l'anticorps polyclonal anti-LG4/5 sur l'adhérence cellulaire à la protéine LG4/5 et à la LN332. Après saturation des puits, une solution d'anticorps anti-LG4/5 (10 μg/ml) a été appliquée pendant une heure avant de réaliser le test d'adhérence avec les cellules HT1080 ($6.10^4$ cellules/puits). (B, C) Effet de la protéine LG4/5 et du peptide KKLRIKSKEK (SEQ ID 1) en solution sur l'adhérence syndécan-1 dépendante au fragment LG4/5. Les cellules HT1080 ont été détachées des boîtes et déposées dans les puits ($6.10^4$ cellules/puits) tel quel ou en présence de la protéine LG4/5 (10 μg/ml) ou du peptide KKLRIKSKEK (SEQ ID 1) (20 μg/ml) comme indiqué sur le graphe. (A, B, C) Les plaques ont été incubées à 37 °C pendant 1 heure. Après lavages, les cellules adhérées ont été fixées et l'adhérence cellulaire a été mesurée comme décrit dans la partie méthodologie. L'adhérence cellulaire en présence de l'anticorps ou des protéines compétitrices a été présentée en pourcentage de l'adhérence cellulaire obtenue sur le même substrat sans compétiteur.

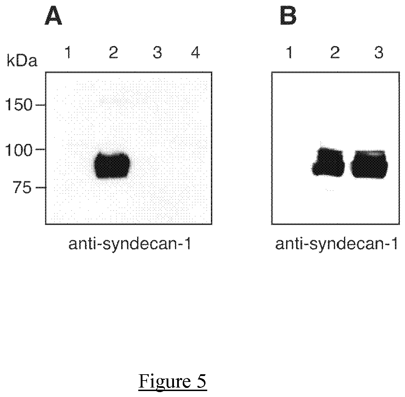

Figure 5 : Mise en évidence de la liaison spécifique du syndécan-1 au peptide KKLRIKSKEK (SEQ ID 1) par chromatographie d'affinité.

(A) Un lysat de kératinocytes humains normaux a été préparé comme décrit dans Matériel et Méthodes et 2 mg de lysat ont été incubé avec des billes de neutravidine non recouvertes (ligne 1) ou recouvertes des peptides KKLRIKSKEK (SEQ ID 1) (ligne 2), PSGKPKSLP (SEQ ID 19) (ligne 3) et TSVTPKQSL (SEQ ID 20) (ligne 4) biotinylés. Après des lavages, le matériel lié aux billes a été digéré avec un mélange d'héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174. (B) Un lysat de kératinocytes humains normaux a été préparé et 2 mg de lysat ont été incubé avec des billes de neutravidine non recouvertes (ligne 1) ou recouvertes de peptide KKLRIKSKEK (SEQ ID 1) biotinylé (ligne 3), ou avec des billes de sépharose recouvertes du fragment LG4/5 entier (ligne 2). Après des lavages, le matériel lié aux billes a été digéré avec un mélange d'héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174. (A, B) La position des marqueurs de poids moléculaire est indiquée sur la gauche.

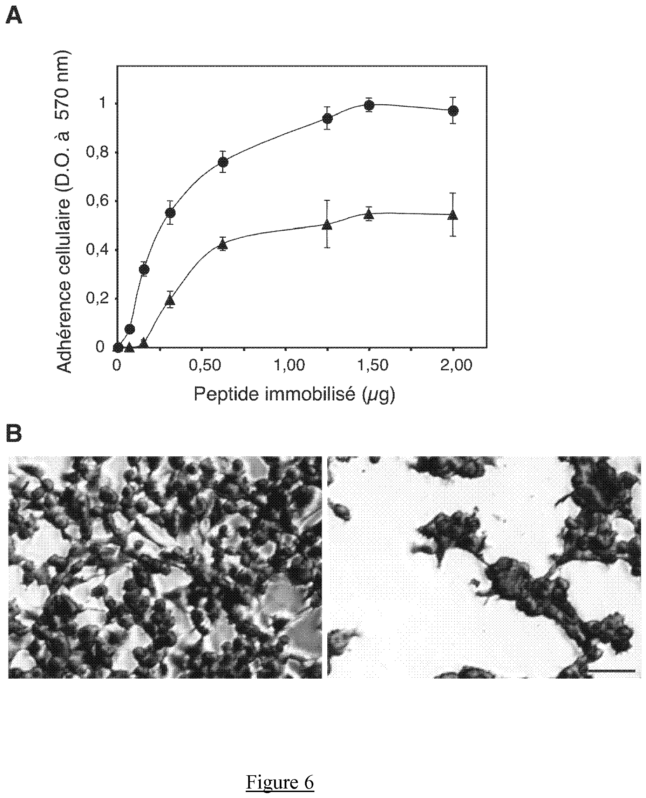

Figure 6 : Adhérence des cellules HT1080 aux peptides KKLRIKSKEK (SEQ ID 1) et NSFMALYLSKGR (SEQ ID 21).

(A) Une gamme décroissante de peptides KKLRIKSKEK (SEQ ID 1) et de peptide NSFMALYLSKGR (SEQ ID 21) ont été immobilisés dans des plaques 96 puits micro Ion et la détermination de la quantité réellement adsorbée a été effectué par un dosage des protéines en se référant à une gamme du peptide correspondant effectuée extemporanément. Les quantités de peptide testées sont indiquées en abscisse sur le graphe. $6.10^4$ cellules ont été déposées dans chaque puits et les plaques ont été incubées à 37 °C pendant 1 heure. Après lavages, les cellules adhérées ont été fixées et l'adhérence cellulaire a été mesurée comme décrit dans la partie méthodologie. (B) Observation des cellules HT1080 adhérés au peptide KKLRIKSKEK (SEQ ID 1) et au peptide NSFMALYLSKGR (SEQ ID 21). L'observation a été faite avec un microscope Axiovert 40 Zeiss équipé d'un bloc interférentiel PlasDic. Barre, 50 $\mu$m.

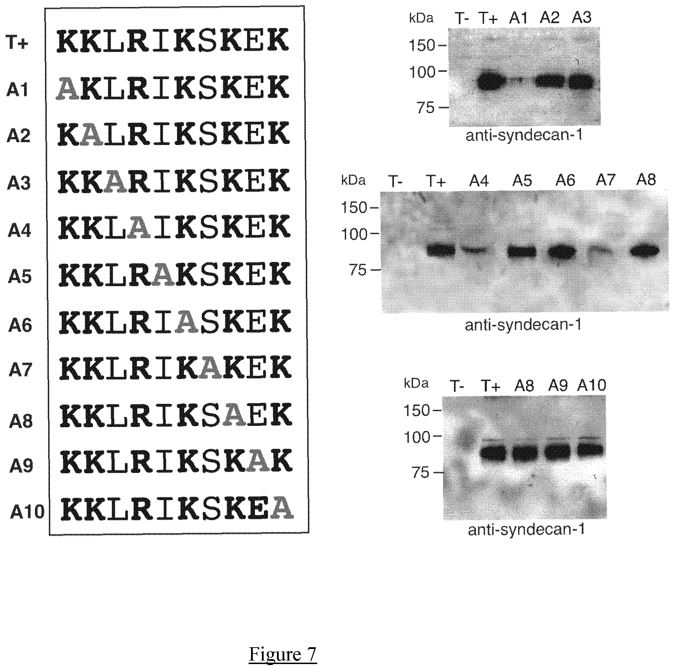

Figure 7 : Mutagénèse du peptide KKLRIKSKEK (SEQ ID 1) et conséquence sur la liaison au syndécan-1.
Une série de mutations ponctuelles ont été introduites dans le peptide KKLRIKSKEK (SEQ ID 1) en remplaçant successivement chaque acide aminé par une alanine (A). Les différents peptides mutés biotinylés sont présentés sur la figure et numérotés de A1 à A10. Le peptide sauvage (T+) est représenté en noir. Les acides aminés « en gras » représentent la position des acides aminés basiques (lysines et arginines) supposés jouer un rôle important dans l'interaction avec les glycosaminoglycannes. Chacune des mutations en alanine (A) est représentée soulignée. Un lysat de kératinocytes humains normaux a été préparé comme décrit dans Matériel et Méthodes et 2 mg de lysat ont été incubé avec des billes de neutravidine non recouvertes (ligne T-), recouvertes du peptides KKLRIKSKEK (SEQ ID 1) (T+) ou recouvertes des différents peptides mutés (A1 à A10). Après des lavages, le matériel lié aux billes a été digéré avec un mélange d' héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174. La position des marqueurs de poids moléculaire est indiquée sur la gauche.

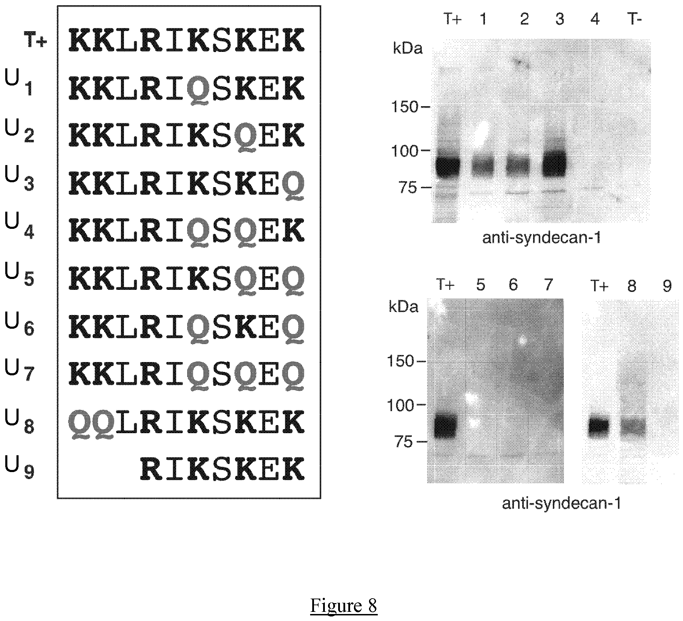

Figure 8 : Mutagénèse du peptide KKLRIKSKEK (SEQ ID 1) et conséquence sur la liaison au syndécan-1.
Une série de mutations ponctuelles ou groupées ont été introduites dans le peptide KKLRIKSKEK (SEQ ID 1) en remplaçant une ou plusieurs Lysine (K) par une glutamine (Q). Les différent peptides mutés biotinylés sont présentés sur la figure et dénommés U1 à U9. Le peptide sauvage est désigné T+. Les acides aminés « en gras » représentent la position des acides aminés basiques (lysines et arginines) supposés jouer un rôle important dans l'interaction avec les glycosaminoglycannes. Chacune des mutations en glutamine (Q), ponctuelle ou multiple, est représentée soulignée. Un peptide raccourci et privé de 3 résidus est désigné U9. Un lysat de kératinocytes humains normaux a été préparé comme décrit dans Matériel et Méthodes et 2 mg de lysat ont été incubé avec des billes de neutravidine non recouvertes (ligne T-), recouvertes du peptides KKLRIKSKEK (T+) ou recouvertes des différents peptides modifiés (U1 à U9). Après des lavages, le matériel lié aux billes a été digéré avec un mélange d'héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174. La position des marqueurs de poids moléculaire est indiquée sur la gauche.

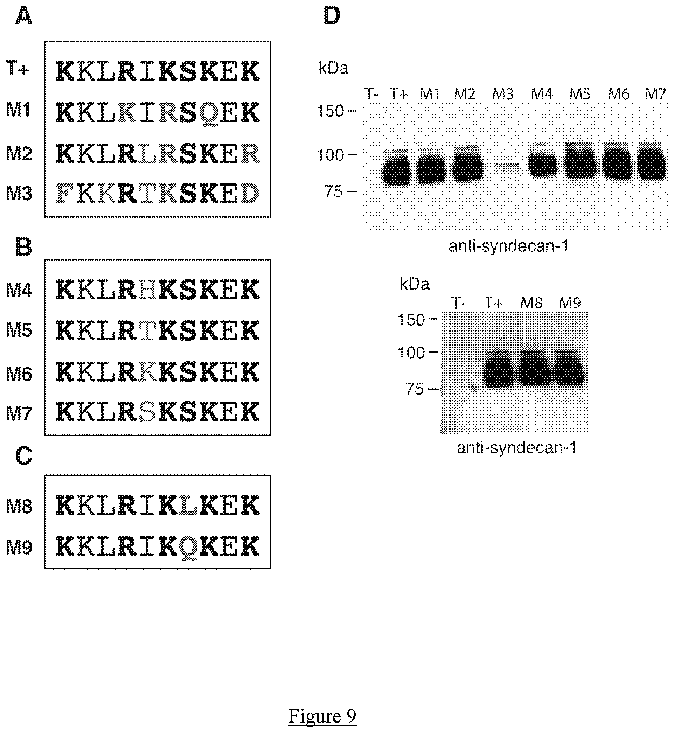

Figure 9 : Mutagénèse du peptide KKLRIKSKEK (SEQ ID 1) par recherche d'homologie dans les protéines de la matrice extracellulaire et conséquence sur la liaison au syndécan-1.
Une série de variants du peptide KKLRIKSKEK (SEQ ID 1) ont été produits sur la base d'homologies de séquences retrouvées dans des protéines de la matrice extracellulaire. Le peptide sauvage est désigné T+. Les acides aminés « en gras » représentent la position des acides aminés jouant un rôle important dans l'interaction avec le syndécan-1 (voir figures 6 et 7). Les acides aminés qui diffèrent de la séquence d'origine sont soulignés. (A) Des séquences homologues de la séquence KKLRIKSKEK (SEQ ID 1) ont été retrouvées dans les protéines de la matrice extracellulaire et ont été appelées M1 (issue de la chaîne de laminine alpha4 humaine ou murine), M2 (issue de la chaîne de laminine alpha3 murine) et M3 (issue de la protéine ADAM 20 humaine). (B) Des séquences homologues de la séquence R-X-KSK ont été retrouvées dans les protéines de la matrice extracellulaire et ont été listées de M4 à M7. (C) Des séquences homologues de la séquence RIK-X-K ont été retrouvées dans les protéines de la matrice extracellulaire et ont été listées de M8 à M9. Des lysats de kératinocytes humains normaux ont été préparés comme décrit dans Matériel et Méthodes et 2 mg de lysat ont été incubé avec des billes de neutravidine non recouvertes (ligne T-), recouvertes du peptides KKLRIKSKEK (SEQ ID 1) (T+) ou recouvertes des différents peptides modifiés (M1 à M9). Après des lavages, le matériel lié aux billes a été digéré avec un mélange d'héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174. La position des marqueurs de poids moléculaire est indiquée sur la gauche.

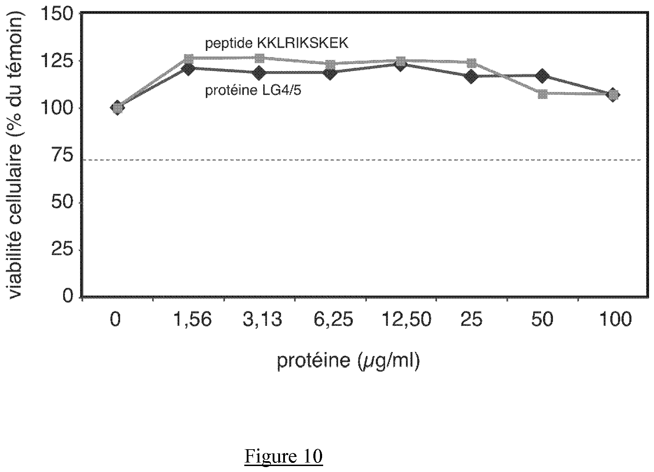

Figure 10 : Effet du peptide KKLRIKSKEK (SEQ ID 1) sur la viabilité des NHKs.

Les NHKs ont été ensemencées dans des plaques 96 puits à raison de $10^4$ cellules par puits. Après 24 heures, les milieux de culture ont été enlevés et remplacés par du milieu KBM-2 contenant les concentrations de peptide KKLRIKSKEK (SEQ ID 1) (courbe du haut) ou de protéine LG4/5 (courbe du bas) indiquées. Après 48 h de contact à 37°C, les milieux ont été enlevés et cette étape a été renouvelée deux fois. Après 48 h de contact à 37°C, les milieux ont été remplacés par le réactif XTT. Les plaques ont ensuite été placées dans un incubateur à 37°C et la lecture de l'absorbance a été effectuée à 6 h. Des témoins sans peptide ni protéine ont été réalisés sur la même plaque.

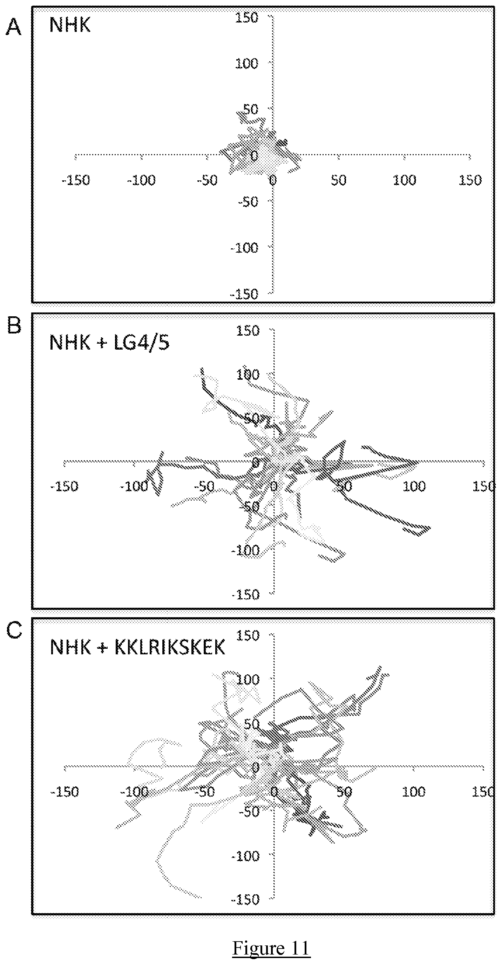

Figure 11 : Effet du peptide KKLRIKSKEK (SEQ ID 1) sur la migration des NHKs par vidéomicroscopie en temps réel sur cellules vivantes.

Les NHKs ont été ensemencées dans des plaques 24 puits à raison de 20 000 cellules par puits en absence (A) ou en présence du fragment LG4/5 (10μg/ml, B) ou du peptide KKLRIKSKEK (SEQ ID 1) (10μg/ml, C) dans du milieu de culture KGM. Le comportement des cellules a ensuite été immédiatement enregistré par vidéomicroscopie en temps réel (Time-Lapse) pendant 5 heures à raison d'une prise d'image toutes les 10 minutes. Les schémas illustrent le comportement de 20 cellules prises au hasard dans un champ donné. Pour faciliter l'analyse des données, les trajets ont été orientées selon x(0), y(0). Les distances parcourues sont indiquées en micromètres sur les axes des graphes.

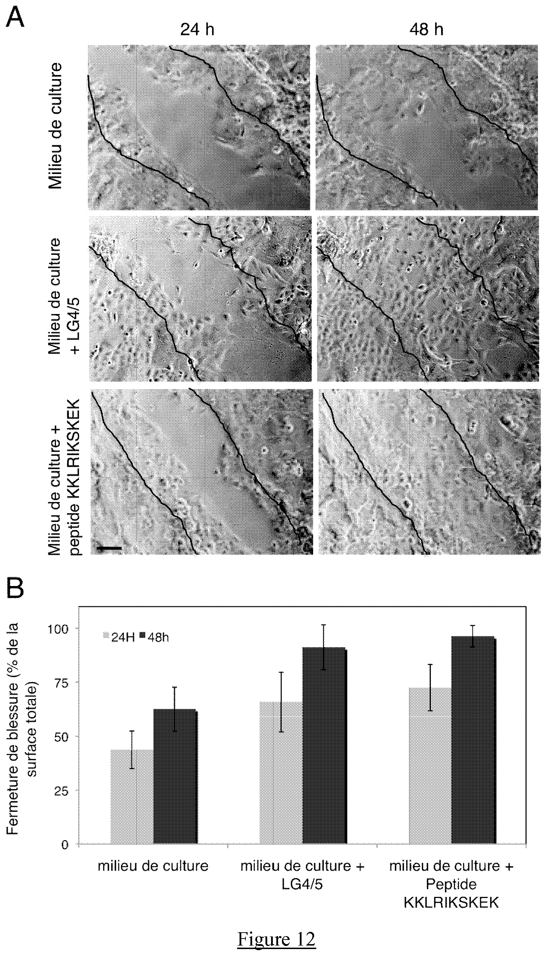

Figure 12 : Effet du peptide KKLRIKSKEK (SEQ ID 1) sur la fermeture de blessure des NHKs.

Les NHKs ont été ensemencées dans des plaques 24 puits et cultivés jusqu'à confluence. Le milieu a été retiré et une blessure a été effectuée dans le tapis cellulaire à l'aide d'un embout de pipette. Après un rinçage avec du PBS, les surfaces ont été incubées avec du milieu de culture KBM-2 à 37°C en absence ou en présence de la protéine LG4/5 (10 μg/ml) ou du peptide KKLRIKSKEK (SEQ ID 1) (20 μg/ml). Le comportement des cellules au niveau de la blessure a ensuite été immédiatement suivi en vidéomicroscopie Time-Lapse à raison d'une image enregistrée toutes les heures sur une période de 48 heures dans une chambre humide contenant 5% CO2. (A) Les images recensées au début de l'expérience, à 24 h et à 48 h ont permis de définir la surface totale de blessure recouverte par les cellules pendant la durée de l'expérience et pour chaque condition (les lignes noires représentent les bords de la plaie au début de l'enregistrement). Barre, 100 μM. (B) Les surfaces blessées (sans cellules) ont été mesurées au début, à 24 h (barres grises) et à 48 h (barres noires) à l'aide du logiciel Adobe Photoshop CS3 Extended (version 10.0) dans les différentes conditions. Le taux de fermeture de chaque blessure a été exprimé en pourcent de la blessure initiale. Chaque condition a été réalisée en tripliquette (3 puits par condition).

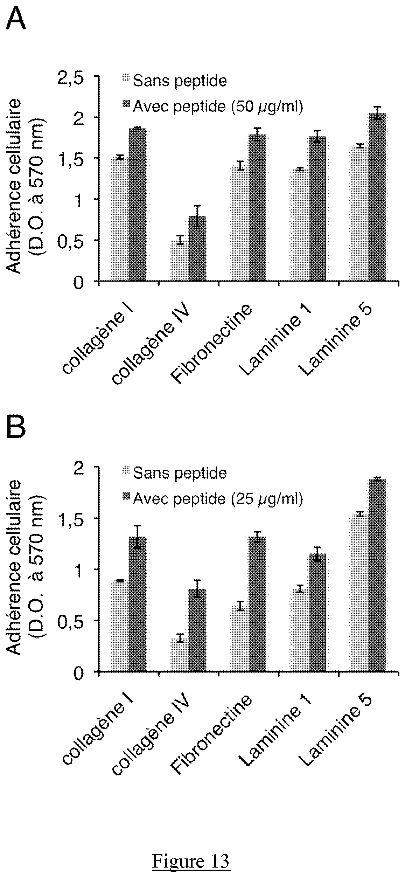

Figure 13 : Analyse de l'effet du peptide KKLRIKSKEK (SEQ ID 1) sur l'adhérence des cellules à différentes protéines de la matrice extracellulaire.

(A) Etude de l'effet du peptide apporté sous forme soluble sur l'adhérence des cellules HT1080. A l'aide d'expériences d'adhérence dose-réponse, une quantité fixe de collagène I (0,02 μg/puits), de collagène IV (0,5 μg/puits), de fibronectine (0,1 μg/puits), de laminine 111 (0,1 μg/puits) et le laminine 332 (0,2 μg/puits) inductrices d'une adhérence moyenne ont été choisies pour cette expérience et immobilisées dans les plaques 96 puits. $6.10^4$ cellules HT1080 ont été déposées dans chaque puits en présence (barres noires) ou en absence (barres grises) du peptide KKLRIKSKEK (SEQ ID 1) (50 μg/ml). Les plaques ont été incubées à 37 °C pendant 1 heure et après lavages, les cellules adhérées ont été fixées. L'adhérence cellulaire a été mesurée comme décrit dans la partie méthodologie.

(B) Etude de l'effet du peptide immobilisé sur l'adhérence des cellules HT1080 aux protéines matricielles. Les protéines matricielles, collagène I (0,02 μg), de collagène IV (0,5 μg), de fibronectine (0,1 μg), de laminine 111 (0,1 μg) et le laminine 332 (0,2 μg) ont été immobilisées seules (barres grises) ou co-immobilisées avec le peptide KKLRIKSKEK (2,5 μg, barres noires) sur des plaques 96 puits. $6.10^4$ cellules HT1080 ont été déposées dans chaque puits et les plaques ont été incubées à 37 °C pendant 1 heure. Après lavages, les cellules adhérées ont été fixées et l'adhérence cellulaire a été mesurée comme décrit dans la partie méthodologie.

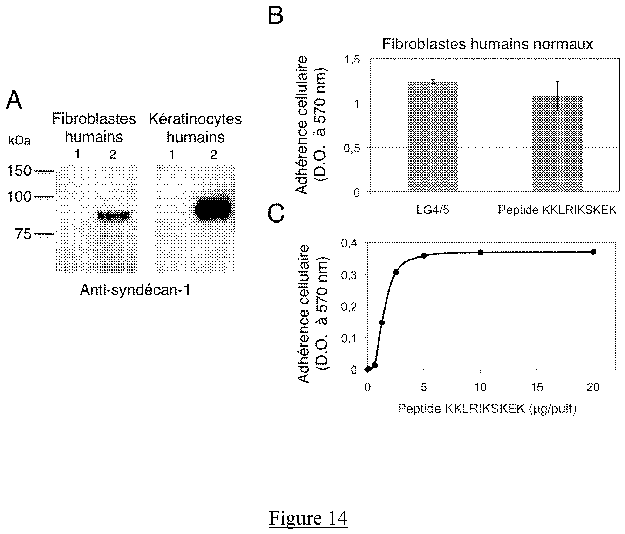

Figure 14: Le syndécan-1 des fibroblastes dermiques interagit avec le peptide KKLRIKSKEK (SEQ ID 1).

(A) Mise en évidence de la liaison du syndécan-1 au peptide KKLRIKSKEK (SEQ ID 1). Un lysat de fibroblastes humains normaux et un lysat de kératinocytes humains normaux ont été préparés comme décrit dans Matériel et Méthodes et 2 mg de chaque lysat ont été incubés avec des billes de neutravidine non recouvertes (lignes 1) ou recouvertes des peptides KKLRIKSKEK (SEQ ID 1) biotinylé (lignes 2). Le contrôle positif réalisé avec le lysat de kératinocytes a été effectué afin de comparer la taille et la quantité du syndécan-1 dans les 2 types cellulaires. Après des lavages, le matériel lié aux billes a été digéré avec un mélange d'héparitinase I et de chondroitinase ABC pour libérer la partie protéique du syndécan-1 des glycosaminoglycannes. Les échantillons ont ensuite été analysés par électrophorèse SDS PAGE dans un gel à 8% d'acrylamide en conditions non réductrices puis analysées par immunotransfert avec l'anticorps polyclonal anti-syndécan-1 H174. La position des marqueurs de poids moléculaire est indiquée sur la gauche. (B, C) Adhérence cellulaire des fibroblastes humains normaux au peptide KKLRIKSKEK (SEQ ID 1) et à la protéine LG4/5. Dans un cas (B), 5 $\mu$g de peptide KKLRIKSKEK et 1 $\mu$g de protéine LG4/5 ont été immobilisés sur une plaque 96 puits et dans l'autre (C), le peptide KKLRIKSKEK a été immobilisé aux concentrations indiquées en abscisse. $6.10^4$ fibroblastes dermiques ont été déposés dans chaque puits et les plaques ont été incubées à 37 °C pendant 1 heure. Après lavages, les cellules adhérées ont été fixées et l'adhérence cellulaire a été mesurée comme décrit dans la partie méthodologie.

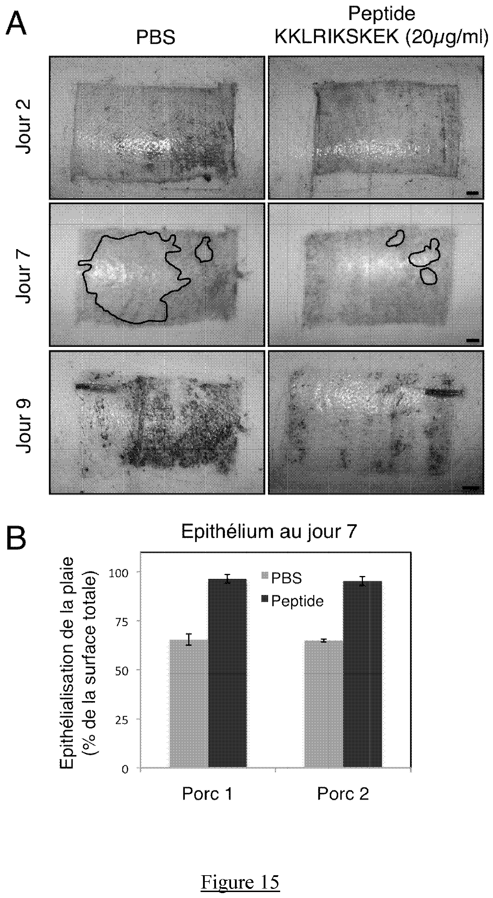

Figure 15 : Efficacité du peptide KKLRIKSKEK (SEQ ID 1) dans l'épithélialisation de plaies cutanées superficielles dans un modèle expérimental chez le porc.

(A) Des plaies superficielles de 0,9 mm d'épaisseur ont été réalisées sur le dos de 2 porcs et ont été traitées par du PBS (témoins) ou par une solution de peptide KKLRIKSKEK à 20$\mu$g/ml, (porc 1) ou 50 $\mu$g/ml (porc 2). L'épithélialisation des plaies évaluée macroscopiquement, montre une différence de recouvrement entre les deux conditions au jour 7. Sur les clichés, les zones non épithélialisées ont été entourées d'une ligne noire. L'observation macroscopique au jour 11 montre une meilleure restauration cutanée en présence du peptide. (B) La surface ré-épithélialisée de chaque plaie photographiée au jour 7 a été évaluée à l'aide du logiciel Adobe Photoshop CS3 Extended et le taux d'épithélialisation a été exprimé en pourcent de la blessure initiale. Ces résultats représentent la moyenne des résultats obtenus après traitement de 4 plaies différentes pour chaque concentration de peptide (2 plaies témoins par condition).

Exemples de réalisation

1°) Procédé de fabrication des peptides

La synthèse peptidique a été effectuée sur un Synthétiseur Milligen 9050 en utilisant une chimie Fmoc-Opfp/Hobt. Le peptide est ensuite détaché de la résine et déprotégé en utilisant une solution de TFA contenant des scavengers (phénol, eau, ethanedithiol et thioanisole). Le peptide est ensuite analysé et purifié sur une colonne Vydac C18, 5mm de 4.6 ou 10mm de diamètre et 250mm de longueur puis caractérisé par spectrométrie de masse électrospray sur un SCIEX API 165. Les peptides biotinylés ont été produits par la société Eurogentec (Eurogentec, Anger, France).

2°) Information sur le peptide d'intérêt : KKLRIKSKEK (SEQ ID 1)

Nombre d'acides aminés: 10
Poids moléculaire: 1257.5
Point isoéléctrique théorique: 10.58
Composition en acides aminés:

| | | |
|---|---|---|
| Arg (R) | 1 | 10.0% |
| Glu (E) | 1 | 10.0% |
| Ile (I) | 1 | 10.0% |
| Leu (L) | 1 | 10.0% |
| Lys (K) | 5 | 50.0% |
| Ser (S) | 1 | 10.0% |

Nombre total de résidus chargés négativement (Asp + Glu): 1
Nombre total de résidus chargés positivement (Arg + Lys): 6
Points isoélectriques théoriques des peptides analogues compris entre 10 et 12 :

- A2 : KALRIKSKEK : 10.46
- A3 : KKARIKSKEK : 10.58
- A5 : KKLRAKSKEK : 10.58
- A6 : KKLRIASKEK : 10.46
- A8 : KKLRIKSAEK : 10.46
- A9 : KKLRIKSKAK : 11,39
- A10 : KKLRIKSKEA : 10.46
- M2 : KKLRLRSKER : 11.73
- M4 :KKLRHKSKEK : 10.58
- M5 : KKLRTKSKEK : 10.58
- M6 : KKLRKKSKEK : 10.68
- M7 : KKLRSKSKEK : 10.58
- M8 : KKLRIKLKEK : 10.58
- M9 : KKLRIKQKEK : 10.58
- U1 : KKLRIQSKEK : 10.46
- U2 : KKLRIKSQEK : 10.46
- U3 : KKLRIKSKEQ : 10.46

3°) Les cellules utilisées pour l'étude

A- les lignées

Les cellules utilisées proviennent de lignées exprimant une quantité importante de syndécan-1 à leur surface (Okamoto *et al.,* 2003). Il s'agit de cellules de la lignée HT1080 (fibrosarcoma, Human), American Type Culture Collection CCL-121.
Ces cellules ont été maintenues en culture dans du milieu DMEM supplémenté avec 10% de sérum de veau foetal et 2mM glutamine et ont été cultivés à 37°C dans un incubateur à CO2 (5 % de CO2, 95% d'air et 98% d'humidité).

B- les kératinocytes primaires

Des kératinocytes humains normaux fraîchement isolés ont été utilisés. Les kératinocytes humains ont été obtenus à partir de biopsie de prépuce (déchet opératoire, Pavillon T-Bis, Hôpital Edouard Herriot). Le milieu de culture utilisé a été le milieu défini pour culture de kératinocytes KGM-2 (contenant : Extrait pituitaire bovin 35 mg, hEGF 10 ng/ml, insuline 5 $\mu$g/ml, Hydrocortisone 0,5 $\mu$g/ml, transferrine 0,1%, épinéphrine 0,1%) fabriqué par Clonetics et commercialisé par Lonza (Belgique) contenant 0,15 mM de CaCl2, pH 7,2 à 7,4.
Les kératinocytes ont été obtenus selon la technique décrite par Boyce et Ham (Cultivation, frozen storage, and clonal growth of normal human epidermal keratinocytes in serum free-media, Tiss Cult. Meth. 1985, 9 :83-93). Les morceaux de peau, après rinçage soigné dans du tampon PBS contenant des antibiotiques, est débarrassé du tissu graisseux situé sous le derme à l'aide d'instruments stériles. La peau a ensuite été découpée en morceaux de 3 mm$^2$, lesquels ont été placés dans une solution stérile de trypsine à 0,25 % dans du PBS pendant 16 heures à 4°C. La séparation derme / épiderme a été effectuée à l'aide de pinces fines dans une boîte de Pétri contenant du milieu de culture afin d'arrêter l'action enzymatique de la trypsine. Les fragments d'épiderme ont été aspirés et refoulés plusieurs fois avec une pipette pour en détacher les cellules basales libres. La suspension cellulaire ainsi obtenue a été centrifugée 5 min à 1000 tours/min et le culot ainsi obtenu a été suspendu dans un volume connu de KBM-2 pour effectuer un comptage des cellules vivantes à l'aide d'un colorant d'exclusion : le bleu trypan. 3.104 cellules vivantes ont été ensemencées par cm2 sur des boîtes pour culture de tissus de 25 cm2 (Corning, Polylabo, France). Les kératinocytes ont été cultivés à 37°C dans un incubateur à CO2 (5 % de CO2, 95% d'air et 98% d'humidité). Le milieu a été changé tous les deux jours. La sub-culture a eu lieu quand les cellules ont atteint la sub-confluence. Le tapis cellulaire a alors été rincé avec du PBS, puis recouvert d'une solution de Trypsine-EDTA (0,05-0,02 %). Après une courte incubation à 37°C, les cellules se sont détachées du support plastique. Les cellules ont alors été ensemencées dans des boîtes de culture de 75 cm$^2$. La congélation des cellules (3 à 5 millions par ampoule) a été réalisée dans le milieu de culture utilisé, en présence de 10% de Diméthyl sulfoxyde (DMSO) et 20 % de sérum de veau sous un volume de 1 ml.

C- les fibroblastes primaires

Des fibroblastes humains normaux fraîchement isolés ont été utilisés. Ils ont été obtenus à partir des biopsies de prépuce utilisées pour l'obtention des kératinocytes. Le milieu de culture utilisé a été le DMEM supplémenté avec 10% de sérum de veau foetal et 2mM glutamine. Lorsque la séparation derme / épiderme a été effectuée, les fragments de derme ont été placés pendant plusieurs jours, dans des boites de pétri en présence de DMEM supplémenté avec 10% de sérum de veau foetal et 2 mM glutamine à 37°C dans un incubateur à CO2 (5 % de CO2, 95% d'air et 98% d'humidité). Le milieu a été changé tous les deux jours. Lorsque les fibroblastes sont sortis des explants dermiques et ont colonisés la boite de pétri, ils ont été décollés de la boite avec de la trypsine et amplifiés selon les techniques classiques de culture cellulaire.

4 ) Analyse quantitative des propriétés d'adhérence cellulaire du peptide d'intérêt par un test colorimétrique:

- Préparation des substrats d'adhérence

Les peptides, la protéine LG4/5 purifiée (Belin et Rousselle, 2007) et la LN332 purifiée (Rousselle *et al.,* 1991) ont été utilisés au cours des expériences d'adhérence cellulaire. Une gamme de 7 concentrations décroissantes a été réalisée par dilution successive dans du PBS (Phosphate Buffer Saline, $KH_2PO_4$ 1,54 mM ; $Na_2HPO_4$ 1,42 mM ; NaCl 131 mM) ou du tampon d'immobilisation $Na_2CO_3$ 20 mM pH 9, à partir d'une solution de départ à 1 mg/ml. Ces solutions ont été immédiatement distribuées sur des plaques de culture 96 puits (Greiner, Dutscher, Brumath, France) à raison de 100 $\mu$l par puits. Les autres protéines matricielles, collagène I bovin (Symatèse Biomatériaux), collagène IV humain, fibronectine et laminine 1 humaines (BD Biosciences, Le Pont de Claix, France) ont été préparées dans du PBS et distribuées sur des plaques 96 puits (Corning, Amsterdam, Pays Bas). Les plaques ont ensuite été placées à +4°C pendant 16 à 18 heures. Les solutions ont ensuite été enlevées par retournement des plaques et chaque puits a été saturé par une solution de PBS-SAB 1% (sérum albumine bovine). Trois puits supplémentaires sans substrat ont subit le même traitement et ont servi de blanc.

Les expériences de comparaison de l'adhérence induites par les peptides KKLRIKSKEK (SEQ ID 1) et NSFMALYLSKGR (SEQ ID 21) a été réalisée en utilisant les plaques 96 puits Microlon (Greiner, Dutscher) sur lesquelles les 2 peptides ont été immobilisés avec la même efficacité. Le dosage de la quantité de peptide immobilisé a été effectué par l'acide Bicinchoninic (BCA Protein Assay, Perbio Science, Brebière, France).

- Test d'adhérence cellulaire

Les cellules ont été détachées des boîtes de culture par une solution de PBS/EDTA 10 mM, puis ont été suspendues dans du milieu DMEM sans additifs pour les lignées cellulaire et KBM-2 sans additifs pour les kératinocytes humains. Le nombre de cellules semées par puit est indiqué sur les graphes ($5.10^4$ à $10^5$ cellules par puits).

- Evaluation du test d'adhérence cellulaire

Après ensemencement des cellules, les plaques multipuits ont été placées dans un incubateur à 37°C sous atmosphère 5 % CO2. Après une incubation de 30 à 60 minutes, les cellules sont observées au microscope contraste de phase afin de vérifier que le test se soit correctement déroulé. Le milieu d'adhérence a alors été éliminé et chaque puits a été lavé avec une solution de PBS stérile afin d'enlever les cellules n'ayant pas adhérées. Les cellules restantes, adhérentes au substrat, ont ensuite été fixées à l'aide d'une solution de glutaraldéhyde 1 % dans du PBS, pendant 15 minutes. La solution de glutaraldéhyde a été enlevée et les cellules ont alors été colorées avec une solution de crystal violet dilué à 1 % dans de l'eau distillée pendant 30 minutes. Après d'importants rinçages à l'eau, les cellules ont été perméabilisées par une solution de triton à 0,02 % pendant 15 minutes, afin de solubiliser le crystal violet. La lecture de l'absorbance a été effectuée à 570 nm, à l'aide d'un lecteur de plaques ELISA reader (MR500, Dynatech, Guernsey Chanel, Island). Chaque point expérimental a été réalisé en trois exemplaires. La valeur du blanc représente la moyenne de l'absorbance des 3 puits témoins (SAB). Celle-ci a été soustraite à chacune des valeurs de densité optique obtenues pour les points expérimentaux. On a ensuite calculé les moyennes des trois valeurs d'absorbances pour chacune des tripliquettes.

Les résultats ont été représentés sous forme de courbe, avec en ordonnée, les valeurs de l'absorbance, et en abscisse, les différentes concentrations en substrat. Les cellules adhérées ont été photographiées en microscopie à contraste de phase.

5 ) Tests d'inhibition de l'adhérence des cellules par compétition

Les plaques 96 puits ont été recouvertes des quantités indiquées de peptide KKLRIKSKEK ou de laminine 332 par adsorption à +4C pendant 16 à 18 heures. Comme précédemment, les puits ont été saturés par une solution de PBS-SAB 1% pendant une heure. Les cellules HT1080 ont été détachées des boîtes de culture puis ont été suspendues dans du milieu DMEM sans sérum. Après comptage des cellules, la protéine LG4/5 entière ou le peptide ont été ajoutés à la suspension cellulaire 30 minutes avant le test d'adhérence. Un contrôle sans compétiteur a systématiquement été effectué et a été considéré comme le 100 % d'adhérence. Dans le cas de l'inhibition par l'anticorps anti-LG4/5, ce dernier a été appliqué sur les protéines immobilisées pendant une heure après la saturation par la solution de PBS-SAB 1%.

6 ) Test de viabilité cellulaire

L'effet du peptide sur la viabilité cellulaire a été analysé à l'aide d'un test colorimétrique (Cell Proliferation Kit XTT, Roche Diagnostics, Meylan, France) sur les kératinocytes humains normaux. Un contrôle avec la protéine LG4/5 entière a été effectué en parallèle. La réaction chimique du test est basée sur la production de NADPH des cellules vivantes permettant la réduction des sels de tétrazolium XTT jaunes en sels de formazan orange. La mesure de l'absorbance est effectuée à 490 nm à l'aide d'un lecteur de plaques ELISA-Reader. Les cellules ont été ensemencées dans des plaques 96 puits à raison de 10 000 cellules par puits (6 puits/condition) dans du milieu de culture KBM-2. Après 24 heures de culture à 37°C en présence de 5% de $CO_2$, les milieux de culture ont été enlevés et remplacés par du milieu sans sérum contenant les quantités de peptide ou de protéine indiquées sur les graphes et le réactif du test. Les plaques ont ensuite été placées dans un incubateur à 37°C et des lectures de l'absorbance ont été effectuées à 1h, 2h, 3h, 4h et 5h. Des témoins sans peptide ont été réalisés sur la même plaque. Les résultats sont présentés sous la forme du pourcentage de la viabilité des cellules mises en présence du peptide ou protéine par rapport aux témoins sans peptide ni protéine. Dans ce cas, la viabilité cellulaire a été calculée selon la formule :

$$\%\text{viabilité} = (\text{Abs. cellules avec peptide} / \text{Abs. cellules témoins}) \times 100.$$

7 ) Expérience de chromatographie d'affinité et détection du syndécan-1

Pour identifier la nature du récepteur cellulaire se liant au peptide KKLRIKSKEK (SEQ ID 1), des expériences de chromatographie d'affinité ou « pull-down » ont été effectuées par l'incubation de billes recouvertes de peptides avec des lysats cellulaires. Les lysats de kératinocytes ou de fibroblastes primaires ont été réalisés à l'aide d'un tampon de lyse 1% triton X-100 en PBS, pH 7.5, contenant N-éthylmaléimide et phénylméthylsulfonylfluoride 50 mM. Le peptide sauvage KKLRIKSKEK (SEQ ID 1) et les peptides mutés ont été couplés à la biotine au niveau de leur extrémité amino-terminale et 100 $\mu$g de chaque peptide biotinylé a été associé à des billes d'agarose-neutravidine (Perbio Science, Bezons, France) par une incubation de 16 heures à +4°C. Les billes ont ensuite été incubées avec 2 mg de lysat cellulaire pendant 2 heures à +4°C. Après 3 lavages avec du tampon de lyse, les billes ont été transférées dans un tampon de digestion (20 mM sodium acetate, 5 mM $CaCl_2$, pH 7.0) contenant 8 mU/ml d'héparitinase I and 50 mU/ml de chondroitinase ABC (Seikagaku America, Coger, Paris, France) pendant 2 heures à 25°C. Ce traitement permet de dénuder la partie protéique du syndécan-1 des glycosaminoglycannes, la rendant plus facilement analysable par immunotransfert. Les échantillons ont ensuite été analysés par électrophorèse SDS PAGE dans un gel à 8% d'acrylamide en conditions non réductrices puis analysées par immunotransfert avec l'anticorps polyclonal anti-syndécan H174 (Santa Cruz Biotechnology, Le Perray en Yvelines, France).

8 ) Vidéomicroscopie en temps réel sur cellules vivantes (Time Lapse)

Les expériences de vidéomicroscopie ont été réalisées avec un microscope Axiovert 100M Zeiss équipé d'une caméra CCD (1 image toutes les 10 minutes ou toutes les heures). Dans les expériences de la figure 11, nous avons analysé les mouvements de 100 cellules à l'aide du logiciel Metaview (Roper Scientific, Princeton Instruments, Evry, France).

9) Cicatrisation *in vivo*

Des plaies cutanées de longueur 12 cm, largeur 8 cm et de profondeur 0,9 mm ont été effectuées à l'aide d'un dermatome sur le dos de 2 porcs charcutier femelles d'environ 4 mois (2 plaies témoin et 4 plaies traitées par porc, de part et d'autre de la colonne vertébrale). Deux concentrations de peptide, 20 $\mu$g/ml (porc 1) et 50 $\mu$g/ml (porc 2) et une solution de PBS (témoins) ont été appliquées sur les plaies le premier jour et à chaque renouvellement du pansement (J1, J2, J4, J7 et J11). L'analyse clinique a été suivie par la prise de photos et des biopsies cutanées ont été réalisées. L'évaluation de la zone épithélialisée a été effectuée sur les photos du jour 7 à l'aide du logiciel Adobe Photoshop CS3 Extended (version 10.0). L'expérience a été effectuée à L'institut Claude Bourgelat Campus Vétérinaire de Lyon (VetAgro Sup) et le protocole a été approuvé par le comité d'éthique du campus vétérinaire de VetAgro Sup.

Résultats :

a/ Etude de l'adhérence des kératinocytes humains normaux et des cellules HT1080 au fragment LG4/5 et au peptide KKLRIKSKEK (SEQ ID 1)

Comme le montre la figure 3, le peptide KKLRIKSKEK (SEQ ID 1) induit l'adhérence cellulaire des cellules HT1080 et des kératinocytes humains normaux de façon dose dépendante. Une expérience contrôle d'adhérence a été effectuée avec les mêmes cellules sur le fragment LG4/5 entier purifié afin de mettre en évidence l'adhérence syndécan-1 dépendante et de comparer la morphologie cellulaire obtenue entre le fragment LG4/5 entier et la séquence peptidique d'interaction du syndécan-1. Les cellules sont solidement ancrées sur le peptide puisqu'elles ont résisté, de façon comparable au fragment LG4/5 entier, aux différents lavages avant fixation. L'adhérence cellulaire maximale a été obtenue lorsqu'une quantité moyenne de 10 microgrammes de peptide a été déposée dans les puits pour l'immobilisation. Le profil d'adhérence des deux types cellulaires HT1080 et NHK est identique sur le peptide d'intérêt. Cette expérience est qualitative et a été réalisée dans le but d'analyser le comportement des cellules au contact du peptide. Les concentrations inductrices de l'adhérence sont donc indicatives puisque (1) elles ne tiennent pas compte de la quantité de peptide réellement immobilisé dans les puits et (2) elles dépendent de la quantité de cellules apportée dans chaque puits. Ces observations indiquent que les quantités annoncées sont surévaluées et que des quantités moins importantes pourront avoir un effet d'induction de l'adhérence cellulaire syndécan-1 dépendante. En effet, un nombre relativement faible de cellules a volontairement été utilisé dans cette expérience dans le but de mettre en évidence l'effet dose-réponse et de pouvoir analyser la morphologie de cellules individuelles. Un tapis trop confluent aurait gêné cette observation. Les photos obtenues en microscopie à contraste de phase montrent que les cellules HT1080 et les kératinocytes adhérés au peptide KKLRIKSKEK (SEQ ID 1) présentent la même morphologie que ces mêmes cellules adhérées au fragment LG4/5 entier. Ceci indique que le peptide KKLRIKSKEK (SEQ ID 1) est capable d'induire les propriétés d'adhérence portées par le fragment LG4/5 entier. Comme décrit largement dans les publications du Demandeur, l'adhérence à LG4/5 induit l'étalement cellulaire sous la forme de prolongements cytoplasmiques protrusifs de type filopodes (Décline *et al.,* 2003, Okamoto *et al.,* 2003, Bachy *et al.*, 2008, Sulka *et al.,* 2009). Ces structures associées au cytosquelette d'actine interviennent dans les processus de migration cellulaire.

Pour confirmer que le peptide KKLRIKSKEK (SEQ ID 1) correspond à la séquence responsable de l'adhérence cellulaire sur le fragment LG4/5, des expériences de compétition ont été effectuées en pré-incubant les cellules avec le peptide KKLRIKSKEK (SEQ ID 1) avant de les faire adhérer au fragment LG4/5 (Figure 4). Dans cette expérience, l'adhérence cellulaire a également été testée sur la LN332 afin de nous assurer de la spécificité de l'effet du peptide sur la protéine LG4/5 et non sur d'autres protéines matricielles. Un contrôle positif et spécifique de l'inhibition de l'adhérence à LG4/5 a été effectué avec un anticorps polyclonal anti-LG4/5 que nous avons caractérisé comme bloquant la fonction de LG4/5. La protéine LG4/5 entière a également été utilisé comme contrôle positif dans cette expérience. Ainsi, les cellules HT1080 ont, soit été utilisées tel quel, soit été pré-incubées avec la protéine LG4/5 entière ou avec le peptide. Elles ont été ensuite déposées sur les substrats LG4/5 et LN332 pour l'expérience d'adhérence. De façon attendue, notre anticorps anti-LG4/5 a inhibé l'adhérence au fragment LG4/5 sans altérer l'adhérence à la LN332. Le fragment LG4/5 apporté en solution a bloqué lui aussi, en se fixant sur le syndécan-1 des cellules, l'adhérence au fragment LG4/5 immobilisé sans perturber l'adhérence à la LN332. Le peptide KKLRIKSKEK (SEQ ID 1) apporté en solution a, lui aussi, bloqué complètement l'adhérence des cellules au fragment LG4/5 et il n'a pas affecté l'adhérence à la LN332. Ce résultat montre que le peptide a été capable à lui seul de se fixer sur le récepteur impliqué dans l'adhérence au fragment LG4/5. Le fait qu'il n'ait pas inhibé l'adhérence à la LN332 confirme la spécificité de son activité sur le fragment LG4/5. Ces résultats montrent que la séquence KKLRIKSKEK (SEQ ID 1) représente la séquence responsable de l'adhérence syndécan-1 dépendante sur le fragment LG4/5. Ils montrent également que le fragment LG4/5 ou le peptide KKLRIKSKEK (SEQ ID 1) apportés sous forme soluble, présentent la capacité d'interagir avec les cellules et de se fixer sur le récepteur syndécan-1. Ceci indique que ces protéines pourraient également activer des voies de signalisation syndécan-1 dépendantes lorsqu'elles sont apportées sous forme soluble.

b/ Mise en évidence de la liaison spécifique du syndécan-1 au peptide KKLRIKSKEK (SEQ ID 1) par chromatographie d'affinité

Pour démontrer que le peptide KKLRIKSKEK (SEQ ID 1) est le site d'interaction du syndécan-1 sur le fragment LG4/5, des expériences de chromatographie d'affinité ont été effectuées en fixant le peptide KKLRIKSKEK (SEQ ID 1) associé à la biotine sur les billes de streptavidine. Le peptide ainsi exposé à la surface des billes sert d'hameçon moléculaire destiné à piéger son récepteur dans un extrait de kératinocytes humains normaux. Dans cette expérience, les 2 autres séquences identifiées dans le fragment LG4/5 comme impliquées dans la liaison à l'héparine (PSGKPKSLP (SEQ ID 19) et TSVTPKQSL (SEQ ID 20), Vives *et al.,* 2003) ont également été biotinylées et testées. Des homogénats de kératinocytes primaires ont été préparés et les billes recouvertes des différents peptides ont été incubées avec des quantités équivalentes de ces lysats (Figure 5A). Les protéines fixées sur les billes ont été ensuite analysées par électrophorèse SDS-PAGE suivi d'une immunodétection pour évaluer leur contenu en syndécan-1. Comme montré figure 5A, seul l'extrait protéique fixé sur les billes recouvertes du peptide KKLRIKSKEK (SEQ ID 1) (ligne 2) présente une protéine reconnue par l'anticorps anti-syndécan-1. Les billes non recouvertes de peptide (témoin négatif, ligne 1), ainsi que les billes recouvertes des deux autres peptides (lignes 3 et 4) n'ont pas retenu le syndécan-1. Pour confirmer l'identité de la protéine associée au peptide KKLRIKSKEK (SEQ ID 1) comme étant le syndécan-1, l'expérience a été répétée en utilisant le fragment LG4/5 entier comme contrôle positif (Figure 5B). Dans ce cas, la protéine purifiée par le peptide KKLRIKSKEK (SEQ ID 1) (ligne 3) correspond bien au syndécan-1, puisque qu'elle correspond à la protéine purifiée par le fragment LG4/5 entier (ligne 2). Les billes non recouvertes de peptide ne montrent aucune fixation non spécifique (ligne 1). Ces résultats montrent que le syndécan-1 est un récepteur spécifique du peptide KKLRIKSKEK (SEQ ID 1) de façon comparable au fragment LG4/5 entier.

c/ Adhérence des cellules HT1080 aux peptides KKLRIKSKEK (SEQ ID 1) et NSFMALYLSKGR (SEQ ID 21) (décrit dans JP2006063033).

Par une approche basée sur la production de peptides recouvrant séquentiellement la totalité de la séquence des modules LG4/5, une autre séquence a été identifiée comme inductrice de l'adhérence cellulaire (Utani et al., 2001 et JP2006063033) via d'autres récepteurs comme le syndécan 2 ou 4. Cette séquence NSFMALYLSKGR (SEQ ID 21) (du résidu 1412 au résidu 1423 de la séquence de la chaine alpha 3) est localisée dans le module LG4. Pour comparer la capacité d'induction de l'adhérence cellulaire du peptide KKLRIKSKEK (SEQ ID 1) avec cette séquence peptidique, un test d'adhérence a été effectué de façon comparative avec les cellules HT1080, qui expriment le syndécan-1 (Figure 6). Les deux peptides ont été immobilisés sur des plaques 96 puits et la quantité de peptide adsorbé a été quantifiée afin de garantir des quantités équivalentes de peptide dans les 2 cas. Une gamme de peptide réellement immobilisé comprise entre 2 µg et 0,07 µg par puits a ainsi été effectuée. Après avoir été détaché des boites de culture dans des conditions préservant les syndécans, les cellules HT1080 ont été déposées dans les puits contenant les deux peptides et sont restés en contact pendant une heure en absence de sérum (Figure 6A). L'analyse quantitative de l'adhérence cellulaire révèle que le peptide KKLRIKSKEK (SEQ ID 1) est plus efficace que le peptide NSFMALYLSKGR (SEQ ID 21) puisque (1) l'adhérence cellulaire est induite pour une quantité de peptide immobilisée plus basse (de l'ordre de 0,025 µg) et (2) l'adhérence cellulaire maximale atteinte au plateau est significativement plus importante (de l'ordre de 200%). Par ailleurs l'analyse de la morphologie cellulaire montre que, si les cellules adhérées à KKLRIKSKEK (SEQ ID 1) présentent une morphologie typique de l'adhérence syndécan-1 dépendante au fragment LG4/5 (voir figure 3), l'adhérence des cellules au peptide NSFMALYLSKGR (SEQ ID 21) n'est pas accompagnée du même étalement cellulaire puisque l'on note l'absence de prolongements cytoplasmiques (Figure 6B). L'adhérence à ce peptide a été décrite comme impliquant les syndécans -2 et -4 (Utani *et al.,* 2001). Ceci suggère que ce peptide pourrait être impliqué dans des processus cellulaires différents de ceux que nous décrivons pour le peptide KKLRIKSKEK (SEQ ID 1).

d/ Mutations

Dans un objectif de caractérisation de l'activité du peptide KKLRIKSKEK (SEQ ID 1), l'importance des différents acides aminés dans l'interaction avec le syndécan-1 a été évaluée en effectuant un certain nombre de substitutions de ces acides aminés. Ce travail a également eu pour objectif d'identifier les résidus essentiels à l'activité du peptide. Dans un premier temps, et pour ne provoquer qu'un minimum d'altérations structurales du peptide, chacun des acides aminés de la séquence KKLRIKSKEK (SEQ ID 1) ont été successivement substitué par un acide aminé neutre, l'alanine (A) (Figure 7). Les expériences de recrutement du syndécan-1 avec cette série de peptides mutés montrent que la substitution individuelle de trois résidus est suffisante pour inhiber l'interaction avec le syndécan-1. Ces résidus sont l'acide aminé 1 : une lysine (K), l'acide aminé 4 : une arginine (R) et l'acide aminé 7 : une sérine (S). On note la présence de 5 résidus Lysine dans la séquence du peptide KKLRIKSKEK (SEQ ID 1), et seule la mutation de la première a provoqué une perte totale de l'activité d'interaction du peptide. Ceci indique que l'activité de chacun des autres résidus Lysine a été compensée lors de l'interaction avec le syndécan-1. Pour caractériser plus précisément le rôle de ces résidus Lysine dans la séquence peptidique, la charge de chacun de ces résidus a été modifiée, et l'impact de ce changement dans la fixation au syndécan-1 a été analysé. Pour cela, ces acides aminés ont été remplacés par une glutamine (Q), acide aminé neutre à pH physiologique (Figure 8). Ces mutations ont été soit ponctuelles (un seul acide aminé substitué), soit regroupées (2 ou 3 substitutions simultanées).

### e/ Séquences homologues

Des recherches d'homologies de la séquence KKLRIKSKEK (SEQ ID 1) dans les protéines de la matrice extracellulaire ont été effectuées afin d'identifier un potentiel homologue ayant des propriétés de liaison au syndécan-1 et de nous assurer de la spécificité de l'activité de cette séquence. Les 4 protéines de la matrice extracellulaire identifiées comme présentant une séquence homologue à la séquence KKLRIKSKEK (SEQ ID 1) de la chaîne de laminine alpha3 humaine sont la chaîne de laminine alpha3 murine (M2), les chaînes de la laminine alpha4 de laminine humaine (M1) et murine (M1) et la protéine transmembranaire ADAM 20 (M3) (Figure 9A). L'ensemble de ces séquences ont été testées pour leur capacité à lier le syndécan-1 et seules les séquences de laminine alpha3 et alpha4 humaines et murines se sont révélées capable de lier le syndécan-1 alors que la séquence de la protéine ADAM 20 a été inactive (Figure 9C). L'ensemble de ces résultats montre que la séquence de liaison au syndécan-1 présente dans la chaîne de laminine alpha3 humaine est conservée chez la souris. De plus, on note qu'une séquence homologue est présente dans la chaîne de laminine alpha4. Le fait que la séquence présente dans la protéine ADAM 20 ne lie pas le syndécan-1 appuie le caractère de spécificité et d'unicité de la séquence présente dans les laminines. Pour étayer les possibilités de substitution de quelques résidus, des recherches d'homologie ont été effectuées sur des fragments de la séquence du peptide et les séquences identifiées ont été replacées dans le cadre du peptide entier (Figure 9B). Notamment, des substitutions de l'acide aminé 5 (Isoleucine, M4 à M7) et de l'acide aminé 7 (Sérine, M8 et M9) ont été identifiées. On remarque figure 9C qu'aucune de ces mutations n'a entraîné d'inhibition de l'interaction.

### f/ Viabilité cellulaire

Enfin pour s'assurer que le peptide n'ait pas d'activité toxique envers les kératinocytes, un test de viabilité cellulaire a été effectué en utilisant une gamme de peptide variant de 100 à 1 $\mu$g/ml. Un contrôle avec la protéine LG4/5 entière a été effectué en parallèle (Figure 10). Les kératinocytes ont été ensemencées dans des plaques 96 puits à raison de $10^4$ cellules par puits. Après 24 heures, les milieux de culture ont été enlevés et remplacés par du milieu KBM-2 contenant les quantités de peptide (courbe rose) ou de protéine LG4/5 (courbe bleue) indiquées sur les graphes. Après deux jours à 37°C, cette étape a été renouvelée deux fois et la quantification des cellules vivantes a été effectuée avec le réactif du test XTT. Les résultats, présentés sous la forme du pourcentage de la viabilité des cellules mises en présence du peptide ou de la protéine LG4/5 par rapport aux témoins, montrent que ni le peptide KKLRIKSKEK, ni la protéine LG4/5 ne possède pas d'activité cytotoxique à long terme sur les kératinocytes de l'épiderme.

### g/ Induction de la migration cellulaire

L'effet de l'apport du fragment LG4/5 entier ou de la séquence KKLRIKSKEK (SEQ ID 1) sous forme soluble dans le milieu de culture des kératinocytes humains normaux a été analysé par vidéomicroscopie en temps réel sur cellules vivantes. Dans un premier temps, l'observation des kératinocytes pendant 5 heures a permis de mettre en évidence des différences significatives selon qu'ils soient en présence ou absence du peptide. Les kératinocytes normaux maintenus dans le milieu de culture classique ont effectués des mouvements circulaires permanents de faible amplitude dont la distance totale ne dépassait jamais 40 $\mu$m (Figure 11A). L'ajout de la protéine LG4/5 ou du peptide KKLRIKSKEK (SEQ ID 1) dans le milieu de culture a induit un changement drastique de comportement (figure 11B et C). Dans les deux cas, les mouvements circulaires des kératinocytes ont été remplacés par des déplacements linéaires aléatoires dont la distance (par rapport à l'origine) pouvait atteindre 150 $\mu$m. Ces résultats montrent que le fragment LG4/5 induit la migration cellulaire lorsqu'il se fixe sur les cellules. Le peptide KKLRIKSKEK (SEQ ID 1) est capable de reproduire l'activité du fragment LG4/5. Si l'adhésion au fragment LG4/5 joue un rôle important dans la migration cellulaire induite par la pre-LN332, ce même fragment LG4/5 (ou sa séquence minima KKLRIKSKEK (SEQ ID 1)) est capable à lui seul d'induire la migration cellulaire.

Dans un second temps, l'effet du fragment LG4/5 et du peptide a été analysé sur la fermeture d'une blessure effectuée au centre d'un tapis de kératinocytes confluents (Figure 12). Comme dans les blessures *in vivo,* la fermeture s'effectue dans ce modèle, par la migration et la prolifération des kératinocytes localisées au niveau des bords de la plaie. Dans les conditions contrôles (milieu de culture seul) la blessure s'est fermée progressivement et les mesures ont montré que les cellules avaient recouvert 43 % de la surface blessée après 24 h et 62 % après 48 h. Lorsque la protéine LG4/5 à été ajoutée au milieu de culture, les cellules ont recouvert 65 % de la surface à 24 h et 91 % à 48 h, ce qui montre que ce domaine de la laminine 332 accélère le processus de fermeture de blessure. Cet effet a été retrouvé voire amplifié avec le peptide KKLRIKSKEK (SEQ ID 1) puisque la fermeture de blessure à 24 h a été de 72 % à 24 h et de 96 % à 48 h (Figure 12A et B). Le peptide présente des propriétés d'induction de la cicatrisation cutanée.

L'hypothèse selon laquelle le peptide présente des propriétés d'induction de la cicatrisation cutanée a été vérifiée par l'étude de l'efficacité du peptide dans l'épithélialisation de plaies cutanées superficielles dans un modèle expérimental chez le porc (Figure 15). Le tissu cutané du porc présente des caractéristiques proches de celle de la peau humaine. L'application du peptide sur des plaies de 0,9 mm de profondeur a favorisé la fermeture de la blessure (Figure 15A). En effet, dans les conditions contrôles (utilisation de PBS), la blessure s'est fermée progressivement et les mesures ont montré que l'épithélium avait recouvert 65 % de la surface blessée 7 jours après la blessure. Lorsque la plaie a été traitée avec le peptide (20 µg/ml ou 50 µg/ml), le pourcentage de ré-épithélialisation de la plaie a été quasiment total au septième jour puisque qu'il a atteint 96 % de la surface totale. Cette expérience montre que le peptide accélère le processus de fermeture des plaies *in vivo.* L'observation macroscopique des plaies après fermeture totale (jour 11, Figure 15 A) révèle, après traitement par le peptide, un tissu cutané homogène et sain présentant des propriétés identiques au tissu avoisinant la zone réparée, alors qu'il apparaît encore fragile et contusionné dans le cas du témoin. Ces résultats indiquent que la peau réparée semble plus solide et de meilleure qualité lorsqu'elle a été traitée par le peptide.

h/ Effet du peptide sur l'adhérence cellulaire à la matrice extracellulaire

Dans le but d'analyser l'effet du peptide KKLRIKSKEK (SEQ ID 1) dans le contexte de la matrice extracellulaire cutanée, nous avons effectué des expériences d'adhérence à ces protéines matricielles en présence du peptide apporté soit sous forme soluble soit co-immobilisé avec ces protéines. Les protéines matricielles testées (collagène I, collagène IV, fibronectine, laminine 111 et laminine 332) sont largement connues pour induire l'adhérence des cellules cutanées par le biais de récepteurs de la famille des intégrines. Pour analyser l'effet du peptide sur l'adhérence cellulaire à ces protéines matricielles, le peptide KKLRIKSKEK (SEQ ID 1) a été mis en contact de cellules HT1080, préalablement dissociées par l'EDTA, avant d'être déposées sur les substrats matriciels (Figure 13A). L'analyse de l'adhérence obtenue par les cellules contrôles (sans peptide, barres grises) et par les cellules traitées par le peptide (barres noires) aux différentes protéines matricielles a permis de mettre en évidence une augmentation de l'adhérence de 20 à 50 % selon les protéines. Cet effet a été encore plus marqué lorsque le peptide a été co-immobilisé avec ces protéines (Figure 13B) puisqu'une fourchette de 20 à 150 % d'augmentation de l'adhérence a été observée. L'ensemble de ces résultats indique que le peptide KKLRIKSKEK (SEQ ID 1) potentialise l'adhérence des cellules HT1080 aux protéines de la matrice extracellulaire. Le peptide KKLRIKSKEK (SEQ ID 1) peut augmenter l'affinité des cellules pour les protéines de la matrice extracellulaire et ainsi permettre une meilleure communication avec le microenvironnement.

i/ Etude de l'adhérence des fibroblastes humains normaux au fragment LG4/5 et au peptide KKLRIKSKEK (SEQ ID 1)

Pour démontrer que les fibroblastes du derme expriment le syndécan-1 et que celui-ci peut interagir avec le peptide KKLRIKSKEK (SEQ ID 1), une expérience de chromatographie d'affinité au peptide KKLRIKSKEK (SEQ ID 1) a été effectuée avec un lysat de fibroblastes humains normaux (Figure 14A). Une expérience contrôle a été effectuée avec des kératinocytes primaires. Les protéines fixées sur les billes ont été ensuite analysées par électrophorèse SDS-PAGE suivi d'une immunodétection pour évaluer leur contenu en syndécan-1. Comme montré figure 14A, le syndécan-1 est exprimé par les fibroblastes et il se lie au peptide KKLRIKSKEK (SEQ ID 1). Les expériences d'adhérence cellulaire montrent que les fibroblastes humains dermiques adhèrent au domaine LG4/5 entier et au peptide KKLRIKSKEK (SEQ ID 1) (Figure 14B). Comme le montre la figure 14C, le peptide KKLRIKSKEK (SEQ ID 1) induit l'adhérence des fibroblastes humains normaux de façon dose dépendante. L'adhérence cellulaire maximale a été obtenue lorsqu'une quantité moyenne de 5 microgrammes de peptide a été déposée dans les puits pour l'immobilisation. Les fibroblastes adhérés au peptide KKLRIKSKEK (SEQ ID 1) et au fragment LG4/5 présentent une morphologie étalée avec présence de prolongements cytoplasmiques (résultats non montrés). Le peptide KKLRIKSKEK (SEQ ID 1) interagit avec les fibroblastes du derme et représente un substrat d'adhérence permettant leur ancrage au microenvironement matriciel et à leur étalement, conditions favorables à la réparation et régénération tissulaires.

**BIBLIOGRAPHIE**

Amano S, Scott IC, Takahara K, Koch M, Champliaud MF, Gerecke DR, Keene DR, Hudson DL, Nishiyama T, Lee S, Greenspan DS, and Burgeson RE. (2000) J. Biol. Chem. 275, 22728-22735.
Aumailley M, Gimond C, and Rousselle P. (1996) Integrin-mediated cellular interactions with laminins. In 'The laminins" Eds. P. Ekblom, R. Timpl, Harwood Academic Publishers, pp 127-158.
Aumailley M, and Rousselle P. (1999) Laminins of the dermo-epidermal junction. Matrix Biol., 18 :19-28.
Bachy S, Letourneur F, and Rousselle P. (2008) Syndecan-1 interaction with the LG4/5 domain in laminin-332 is essential for keratinocyte migration. J Cell Physiol., 214 :238-249.

Baker SE, Hopkinson SB, Fitchmun M, Andreason GL, Frasier F, Plopper G, Quaranta V, and Jones JC. (1996) J. Cell Sci. 109, 2509-2520.

Belin V, and Rousselle P. (2006) Production of a recombinantly expressed laminin fragment by HEK293-EBNA cells cultured in suspension in a dialysis-based bioreactor. Protein Expr. Purif. 48:43-48.

Bernfield M, Gotte M, Park PW, Reizes O, Fitzgerald ML, Lincecum J, and Zako M. (1999) Functions of cell surface heparan sulfate proteoglycans. Ann. Rev. Biochem. 68, 729-777.

Clark RAF. (1996). Wound repair : overview and general considerations. In : The molecular and cellular biology of wound repair. Clark R.A.F, Editor. Plenum Press/New York. 3-50.

Decline F, and Rousselle P. (2001). Keratinocyte migration requires alpha2beta1 integrin-mediated interaction with the laminin 5 gamma2 chain. J. Cell Sci. 114:811-823.

Decline F, Okamoto O, Mallein-Gerin F, Helbert B, Bernaud J, Rigal D, Rousselle P. (2003). Keratinocyte motility induced by TGF-beta1 is accompanied by dramatic changes in cellular interactions with laminin 5. Cell Motil. Cytoskeleton 54:64-80.

Elenius K, Vainio S, Laato M, Salmivirta M, Thesleff I and Jalkanen M. (1991) Induced expression of syndecan in healing wounds. J. Cell Biol. 114:585-595.

Frank DE and Carter WG. (2004). Laminin 5 deposition regulates keratinocyte polarization and persistent migration. J. Cell Sci. 117:1351-1363.

Gallo R, Kim C, Kokenyesi R, Adzick NS and Bernfield M. (1996) Syndecans-1 and -4 are induced during wound repair of neonatal but not fetal skin. J. Invest. Dermatol. 107:676-683.

Ghohestani RF, Li K, Rousselle P, and Uitto J. (2001) Molecular organization of the cutaneous basement membrane zone. Clin. Dermatol., 19:551-562.

Goldfinger LE, Hopkinson SB, deHart GW, Collawn S, Couchman JR, and Jones JC. (1999) The alpha3 laminin subunit, alpha6beta4 and alpha3beta1 integrin coordinately regulate wound healing in cultured epithelial cells and in the skin. J. Cell Sci. 112:2615-2629.

Goldfinger LE, Stack MS, and Jones JC. (1998) Processing of laminin-5 and its functional consequences: role of plasmin and tissue-type plasminogen activator. J. Cell Biol. 141: 255-265.

Hynes RO. (1992) Integrins : versatility, modulation, and signaling in cell adhesion. Cell 69:11-25.

Jaakkola P, Kontusaari S, Kauppi T, Maata A, and Jalkanen M. (1998) Wound reepithelialization activates a growth factor-responsive enhancer in migrating keratinocytes. FASEB J 12: 959-969.

Lampe PD, Nguyen BP, Gil S, Usui M, Olerud J, Takada Y, and Carter WG. (1998) Cellular interaction of integrin alpha3beta1 with laminin 5 promotes gap junctional communication. J. Cell Biol. 143,1735-1747.

Larjava H, Salo T, Haaspasalmi K, Kramer RH, Heino J. (1993) Expression of integrins and basement membrane components by wound keratinocytes. J. Clin. Invest. 92:1425-1435.

Marinkovich MP, Lunstrum GP, and Burgeson RE. (1992) The anchoring filament protein kalinin is synthesized and secreted as a high molecular weight precursor. J. Biol. Chem. 267, 17900-17906.

Masuda R, Mochizuki M, Hozumi K, Takeda A, Uchinuma E, Yamashina S, Nomizu M, Kadoya Y. (2009) A novel cell-adhesive scaffold material for delivering keratinocytes reduces granulation tissue in dermal wounds. Wound Repair Regen. 17:127-135.

Nguyen BP, Ryan MC, Gil SG and Carter WG. (2000) Deposition of laminin 5 in epidermal wounds regulates integrin signaling and adhesion. Curr. Opin. Cell Biol. 12:554-562.

Niessen CM, Hogervorst F, Jaspars LH, de Melker AA, Delwel GO, Hulsman EH, Kuikman I and Sonnenberg A. (1994) The alpha 6 beta 4 integrin is a receptor for both laminin and kalinin. Exp Cell Res 211:360-367.

Okamoto O, Bachy S, Odenthal U, Bernaud J, Rigal D, Lortat-Jacob H, Smyth N and Rousselle P. (2003) Normal human keratinocytes bind to the alpha3LG4/5 domain of unprocessed laminin-5 through the receptor syndecan-1. J. Biol. Chem. 278:44168-44177.

Oksala O, Salo T, Tammi R, Hakkinen L, Jalkanen M, Inki P and Larjava H. (1995) Expression of proteoglycans and hyaluronan during wound healing. J. Histochem. Cytochem. 43:125-135.

Rousselle P, Aumailley M. (1994) Kalinin is more efficient than laminin in promoting adhesion of primary keratinocytes and some other epithelial cells and has a different requirement for integrin receptors. J. Cell Biol. 125: 205-214.

Rousselle P, Keene DR, Champliaud MF, Van der Rest M, and Burgeson R.E. (1997) Laminin 5 binds type VII collagen thru its NC1 domain. J. Cell Biol. 138:719-728.

Rousselle P, Lunstrum GP, Keene DR, and Burgeson RE. (1991) Kalinin : an epithelium-specific basement membrane adhesion molecule that is a component of anchoring filaments. J. Cell Biol. 114:567-576.

Ryan MC, Lee K, Miyashita Y, and Carter WG. (1999) Targeted disruption of the LAMA3 gene in mice reveals abnormalities in survival and late stage differentiation of epithelial cells. J. Cell Biol. 145:1309-1323.

Ryan MC, Tizard R, VanDevanter DR and Carter WG. (1994) Cloning of the LamA3 gene encoding the alpha 3 chain of the adhesive ligand epiligrin. Expression in wound repair. J. Biol. Chem. 269:22779-22787.

Sigle RO, Gil SG, Bhattacharya M, Ryan MC, Yang TM, Brown TA, Boutaud A, Miyashita Y, Olerud J, and Carter WG. 2004. Globular domains 4/5 of the laminin alpha3 chain mediate deposition of precursor laminin 5. J. Cell Sci. 117:4481-4494.

Sonnenberg A, de Melker AA, Martinez de Velasco AM, Janssen H, Calafat J, and Niessen CM. (1993) Formation of hemidesmosomes in cells of a transformed murine mammary tumor cell line and mechanisms involved in adherence of these cells to laminin and kalinin. J. Cell Sci. 106:1083-1102.

Stepp MA, Gibson HE, Gala PH, Iglesia DD, Pajoohesh-Ganji A, Pal-Ghosh S, Brown M, Aquino C, Schwartz AM, Goldberger O, Hinkes MT and Bernfield M. (2002) Defects in keratinocyte activation during wound healing in the syndecan-1-deficient mouse. J. Cell Sci. 115, 4517-4531.

Sulka B, Lortat-Jacob H, Terreux R, Letourneur F, and Rousselle P. (2009) Tyrosine déphosphorylation of the syndecan-1 PDZ binding domain regulates syntenin-1 recruitment. J. Biol. Chem. Feb 19.

Timpl R, Tisi D, Talts JF, Andac Z, Sasaki T, and Hohenester E. (2000) Structure and function of laminin LG modules. Matrix Biol., 19:309-317.

Tisi D, Talts JF, Timpl R, a,d Hohenester E. Structure of the C-terminal laminin G-like domain pair of the laminin alpha2 chain harbouring binding sites for alpha-dystroglycan and heparin. Embo J. 2000, 19: 1432-1440.

Tunggal L, Ravaux J, Pesch M, Smola H, Krieg T, Gaill F, Sasaki T, Timpl R, Mauch, C and Aumailley M. (2002) Defective laminin 5 processing in cylindroma cells. Am. J. Pathol. 160:459-468.

Utani A, Nomizu M, Matsuura H, Kato K, Kobayashi T, Takeda U, Aota S, Nielsen PK and Shinkai H. (2001) A unique sequence of the laminin alpha 3 G domain binds to heparin and promotes cell adhesion through syndecan-2 and -4. J. Biol. Chem. 276:28779-28788.

Vivès R, Crublet E, Andieu JP, Gagnon J, Rousselle P, and Lortat-Jacob H. (2004) A novel strategy for defining critical amino acid residues involved in protein/glycosaminoglycan interactions. J. Biol. Chem., 279:54327-54333.

Woods, A, and Couchman JR. (2000) Integrin modulation by lateral association. J. Biol. Chem. 275, 24233-24236

**Revendications**

1. Peptide synthétique comprenant ou constitué de la séquence $Kaa_2aa_3Raa_5aa_6aa_7aa_8aa_9aa_{10}$, dans laquelle, à pH physiologique :

- $aa_2$, $aa_6$, $aa_8$, $aa_{10}$ sont des résidus de charge positive ; au plus un d'entre eux pouvant être substitué par un résidu de charge neutre,
- $aa_3$ est un résidu de charge neutre,
- $aa_5$ est un résidu de charge neutre,
- $aa_7$ est un résidu de charge neutre à l'exception de l'alanine, et
- $aa_9$ est un résidu de charge négative ou un résidu de charge neutre

la séquence étant apte à se lier au récepteur syndécan-1,
à l'exception des peptides comprenant ou constitué de la séquence KKLRIKSKEK (SEQ ID 1).

2. Peptide selon la revendication 1, *caractérisé en ce qu'*il comprend ou est constitué d'une séquence choisie dans le groupe suivant :

- KALRIKSKEK (SEQ ID 2)
- KKARIKSKEK (SEQ ID 3)
- KKLRAKSKEK (SEQ ID 4)
- KKLRIASKEK (SEQ ID 5)
- KKLRIKSAEK (SEQ ID 6)
- KKLRIKSKAK (SEQ ID 7)
- KKLRIKSKEA (SEQ ID 8)

- KKLRLRSKER (SEQ ID 9)
- KKLRHKSKEK (SEQ ID 10)
- KKLRTKSKEK (SEQ ID 11)
- KKLRKKSKEK (SEQ ID 12)
- KKLRSKSKEK (SEQ ID 13)
- KKLRIKLKEK (SEQ ID 14)
- KKLRIKQKEK (SEQ ID 15)
- KKLRIQSKEK (SEQ ID 16)
- KKLRIKSQEK (SEQ ID 17)
- KKLRIKSKEQ (SEQ ID 18).

**3.** Peptide synthétique

• contenant au plus 30 acides aminés et comprenant ou constitué de la séquence KKLRIKSKEK (SEQ ID 1), ou
• comprenant ou constitué de la séquence $Kaa_2aa_3Raa_5aa_6aa_7aa_8aagaa_{10}$, dans laquelle, à pH physiologique :

- $aa_2$, $aa_6$, $aa_8$, $aa_{10}$ sont des résidus de charge positive ; au plus un d'entre eux pouvant être substitué par un résidu de charge neutre,
- $aa_3$ est un résidu de charge neutre,
- $aa_5$ est un résidu de charge neutre,
- $aa_7$ est un résidu de charge neutre à l'exception de l'alanine, et
- $aa_9$ est un résidu de charge négative ou un résidu de charge neutre,

la séquence étant apte à se lier au récepteur syndécan-1, à l'exception des peptides comprenant ou constitué de la séquence KKLRIKSKEK (SEQ ID 1)
pour utilisation comme médicament.

**4.** Peptide pour son utilisation selon la revendication 3, *caractérisé en ce qu'*il contient ou est constitué d'une séquence choisie dans le groupe suivant :

- KALRIKSKEK (SEQ ID 2)
- KKARIKSKEK (SEQ ID 3)
- KKLRAKSKEK (SEQ ID 4)
- KKLRIASKEK (SEQ ID 5)
- KKLRIKSAEK (SEQ ID 6)
- KKLRIKSKAK (SEQ ID 7)
- KKLRIKSKEA (SEQ ID 8)
- KKLRLRSKER (SEQ ID 9)
- KKLRHKSKEK (SEQ ID 10)
- KKLRTKSKEK (SEQ ID 11)
- KKLRKKSKEK (SEQ ID 12)
- KKLRSKSKEK (SEQ ID 13)
- KKLRIKLKEK (SEQ ID 14)
- KKLRIKQKEK (SEQ ID 15)
- KKLRIQSKEK (SEQ ID 16)
- KKLRIKSQEK (SEQ ID 17)
- KKLRIKSKEQ (SEQ ID 18).

**5.** Peptide pour son utilisation selon l'une des revendications 3 à 4 *caractérisé en ce qu'*il est utilisé dans la cicatrisation de la peau.

**6.** Peptide pour son utilisation selon l'une des revendications 3 à 4 *caractérisé en ce qu'*il est utilisé dans la régénération tissulaire.

**7.** Composition pharmaceutique ou cosmétique comprenant un peptide synthétique, ledit peptide :

• contenant au plus 30 acides aminés et comprenant ou constitué de la séquence KKLRIKSKEK (SEQ ID 1), ou
• comprenant ou constitué de la séquence $Kaa_2aa_3Raa_5aa_6aa_7aa_8aagaa_{10}$, dans laquelle, à pH physiologique :

- aa$_2$, aa$_6$, aa$_8$, aa$_{10}$ sont des résidus de charge positive ; au plus un d'entre eux pouvant être substitué par un résidu de charge neutre,
- aa$_3$ est un résidu de charge neutre,
- aa$_5$ est un résidu de charge neutre,
- aa$_7$ est un résidu de charge neutre à l'exception de l'alanine, et
- aa$_9$ est un résidu de charge négative ou un résidu de charge neutre,

la séquence étant apte à se lier au récepteur syndécan-1, à l'exception des peptides comprenant ou constitué de la séquence KKLRIKSKEK (SEQ ID 1).

8. Composition selon la revendication 7, *caractérisée* **en ce qu'**elle se présente sous la forme d'une crème, d'un hydrogel, d'une solution, d'une formulation injectable ou d'un spray comprenant éventuellement des kératinocytes autologues ou des cellules mésenchymateuses.

9. Composition selon la revendication 7, *caractérisée* **en ce qu'**elle se présente sous la forme d'un support sur lequel est greffé ou déposé, ou à l'intérieur duquel est incorporé le peptide synthétique, le support se présentant sous la forme d'un film ou d'une matrice constitué :

- d'un matériau biologique choisi dans le groupe comprenant le collagène, la gélatine, un polysaccharide, l'acide hyaluronique, la cellulose, la carboxyméthylcellulose, la pectine, le chitosane, du derme humain ou animal acellularisé, ou
- d'un matériau synthétique choisi dans le groupe comprenant le silicone, le polyuréthane, le PLLA, ou
- d'un matériau textile du type pansement choisi dans le groupe comprenant le coton, le polyester, la polyamide.

10. Utilisation d'une composition selon la revendication 7 comme milieu de culture destiné à la culture *in vitro* de cellules épithéliales ou mésenchymateuses.

11. Support de culture de cellules épithéliales comprenant un support se présentant sous la forme d'une boite de culture en polystyrène, d'un film ou d'une matrice poreuse réalisée en biopolymère, d'une membrane textile ou d'un gel de collagène, sur lequel est greffé ou adsorbé un peptide synthétique, ledit peptide :

• contenant au plus 30 acides aminés et comprenant ou constitué de la séquence KKLRIKSKEK (SEQ ID 1), ou
• comprenant ou constitué de la séquence Kaa$_2$aa$_3$Raa$_5$aa$_6$aa$_7$aa$_8$aagaa$_{10}$, dans laquelle, à pH physiologique :

- aa$_2$, aa$_6$, aa$_8$, aa$_{10}$ sont des résidus de charge positive ; au plus un d'entre eux pouvant être substitué par un résidu de charge neutre,
- aa$_3$ est un résidu de charge neutre,
- aa$_5$ est un résidu de charge neutre,
- aa$_7$ est un résidu de charge neutre à l'exception de l'alanine, et
- aa$_9$ est un résidu de charge négative ou un résidu de charge neutre,

la séquence étant apte à se lier au récepteur syndécan-1, à l'exception des peptides comprenant ou constitué de la séquence KKLRIKSKEK (SEQ ID 1).

**Patentansprüche**

1. Synthetisches Peptid, umfassend die oder bestehend aus der Sequenz Kaa$_2$aa$_3$Raa$_5$aa$_6$aa$_7$aa$_8$aa$_9$aa$_{10}$, in der, bei physiologischem pH:

- aa$_2$, aa$_6$, aa$_8$, aa$_{10}$ Reste mit positiver Ladung sind; höchstens einer von diesen durch einen Rest neutraler Ladung ersetzt werden kann,
- aa$_3$ ein Rest mit neutraler Ladung ist,
- aa$_5$ ein Rest mit neutraler Ladung ist,
- aa$_7$ ein Rest mit neutraler Ladung mit Ausnahme von Alanin ist und
- aa$_9$ ein Rest mit negativer Ladung oder ein Rest mit neutraler Ladung ist,

wobei die Sequenz in der Lage ist, sich an den Syndecan-1-Rezeptor zu binden,

mit Ausnahme der Peptiden, die die Sequenz KKLRIKSKEK (SEQ ID 1) umfassen oder daraus bestehen.

2. Peptid gemäß Anspruch 1, *dadurch gekennzeichnet, dass* es eine Sequenz umfasst oder aus ihr besteht, die aus der folgenden Gruppe ausgewählt wird:

- KALRIKSKEK (SEQ ID 2)
- KKARIKSKEK (SEQ ID 3)
- KKLRAKSKEK (SEQ ID 4)
- KKLRIASKEK (SEQ ID 5)
- KKLRIKSAEK (SEQ ID 6)
- KKLRIKSKAK (SEQ ID 7)
- KKLRIKSKEA (SEQ ID 8)
- KKLRLRSKER (SEQ ID 9)
- KKLRHKSKEK (SEQ ID 10)
- KKLRTKSKEK (SEQ ID 11)
- KKLRKKSKEK (SEQ ID 12)
- KKLRSKSKEK (SEQ ID 13)
- KKLRIKLKEK (SEQ ID 14)
- KKLRIKQKEK (SEQ ID 15)
- KKLRIQSKEK (SEQ ID 16)
- KKLRIKSQEK (SEQ ID 17)
- KKLRIKSKEQ (SEQ ID 18).

3. Synthetisches Peptid

• das höchstens 30 Aminosäuren enthält und die Sequenz KKLRIKSKEK (SEQ ID 1) umfasst oder daraus besteht, oder
• die Sequenz $Kaa_2aa_3Raa_5aa_6aa_7aa_8aa_9aa_{10}$ umfasst oder daraus besteht, in der, bei physiologischem pH:

- $aa_2$, $aa_6$, $aa_8$, $aa_{10}$ Reste mit positiver Ladung sind; höchstens einer von diesen durch einen Rest neutraler Ladung ersetzt werden kann,
- $aa_3$ ein Rest mit neutraler Ladung ist,
- $aa_5$ ein Rest mit neutraler Ladung ist,
- $aa_7$ ein Rest mit neutraler Ladung mit Ausnahme von Alanin ist und
- $aa_9$ ein Rest mit negativer Ladung oder ein Rest mit neutraler Ladung ist,

wobei die Sequenz in der Lage ist, sich an den Syndecan-1-Rezeptor zu binden, mit Ausnahme der Peptiden, die die Sequenz KKLRIKSKEK (SEQ ID 1) umfassen oder daraus bestehen, zur Verwendung als Medikament.

4. Peptid zur Verwendung gemäß Anspruch 3, *dadurch gekennzeichnet, dass* es eine Sequenz enthält oder aus ihr besteht, die aus der folgenden Gruppe ausgewählt wird:

- KALRIKSKEK (SEQ ID 2)
- KKARIKSKEK (SEQ ID 3)
- KKLRAKSKEK (SEQ ID 4)
- KKLRIASKEK (SEQ ID 5)
- KKLRIKSAEK (SEQ ID 6)
- KKLRIKSKAK (SEQ ID 7)
- KKLRIKSKEA (SEQ ID 8)
- KKLRLRSKER (SEQ ID 9)
- KKLRHKSKEK (SEQ ID 10)
- KKLRTKSKEK (SEQ ID 11)
- KKLRKKSKEK (SEQ ID 12)
- KKLRSKSKEK (SEQ ID 13)
- KKLRIKLKEK (SEQ ID 14)
- KKLRIKQKEK (SEQ ID 15)
- KKLRIQSKEK (SEQ ID 16)
- KKLRIKSQEK (SEQ ID 17)

- KKLRIKSKEQ (SEQ ID 18).

5. Peptid zur Verwendung gemäß einem der Ansprüche 3 bis 4, *dadurch gekennzeichnet, dass* es zur Wundheilung eingesetzt wird.

6. Peptid zur Verwendung gemäß einem der Ansprüche 3 bis 4, *dadurch gekennzeichnet, dass* es bei der Geweberegeneration eingesetzt wird.

7. Pharmazeutische oder kosmetische Zusammensetzung umfassend ein synthetisches Peptid, wobei dieses Peptid:

   • höchstens 30 Aminosäuren enthält und die Sequenz KKLRIKSKEK (SEQ ID 1) umfasst oder daraus besteht, oder
   • die Sequenz $Kaa_2aa_3Raa_5aa_6aa_7aa_8aa_9aa_{10}$ umfasst oder aus ihr besteht, in der, bei physiologischem pH:

      - $aa_2$, $aa_6$, $aa_8$, $aa_{10}$ Reste mit positiver Ladung sind; höchstens einer von diesen durch einen Rest neutraler Ladung ersetzt werden kann,
      - $aa_3$ ein Rest mit neutraler Ladung ist,
      - $aa_5$ ein Rest mit neutraler Ladung ist,
      - $aa_7$ ein Rest mit neutraler Ladung mit Ausnahme von Alanin ist und
      - $aa_9$ ein Rest mit negativer Ladung oder ein Rest mit neutraler Ladung ist,

   wobei die Sequenz in der Lage ist, sich an den Syndecan-1-Rezeptor zu binden, mit Ausnahme der Peptiden, die die Sequenz KKLRIKSKEK (SEQ ID 1) umfassen oder daraus bestehen.

8. Zusammensetzung gemäß Anspruch 7, *dadurch gekennzeichnet, dass* sie die Form einer Creme, eines Hydrogels, einer Lösung, einer injizierbaren Rezeptur oder eines Sprays eventuell mit autologen Keratinozyten oder Mesenchymalzellen hat.

9. Zusammensetzung gemäß Anspruch 7, *dadurch gekennzeichnet, dass* sie die Form eines Trägers hat, auf den das synthetische Peptid aufgepfropft oder abgeschieden ist, oder worin es integriert ist, wobei dieser Träger die Form eines Films oder einer Matrix hat, bestehend aus:

   - einem biologischen Material, das aus der Gruppe umfassend Collagen, Gelatine, ein Polysaccharid, Hyaluronsäure, Zellulose, Karboxymethylzellulose, Pektin, Chitosan, azellularisierte humane oder tierische Dermis ausgewählt wird, oder
   - einem synthetischen Material, das aus der Gruppe umfassend Silikon, Polyurethan, PLLA ausgewählt wird oder
   - einem textilen Material vom Typ Verband, das aus der Gruppe umfassend Baumwolle, Polyester, Polyamid ausgewählt wird.

10. Verwendung einer Zusammensetzung gemäß Anspruch 7 als Nährmedium, zur *in vitro*-Kultur von Epithel- oder Mesenchymalzellen.

11. Epithelzell- Kulturträger mit einem Träger in Form eines Nährmedium- Behälters aus Polystyrol, eines Films oder einer porösen Matrix aus Biopolymer, einer Textilmembran oder eines Collagengels, auf den ein synthetisches Peptid aufgetragen oder absorbiert ist, wobei dieses Peptid:

   • höchstens 30 Aminosäuren enthält und oder die Sequenz KKLRIKSKEK (SEQ ID 1) umfasst oder daraus besteht, oder
   • die Sequenz $Kaa_2aa_3Raa_5aa_6aa_7aa_8aa_9aa_{10}$ umfasst oder daraus besteht, in der, bei physiologischem pH:

      - $aa_2$, $aa_6$, $aa_8$, $aa_{10}$ Reste mit positiver Ladung sind; höchstens einer von diesen durch einen Rest neutraler Ladung ersetzt werden kann,
      - $aa_3$ ein Rest mit neutraler Ladung ist,
      - $aa_5$ ein Rest mit neutraler Ladung ist,
      - $aa_7$ ein Rest mit neutraler Ladung mit Ausnahme von Alanin ist und
      - $aa_9$ ein Rest mit negativer Ladung oder ein Rest mit neutraler Ladung ist,

   wobei die Sequenz in der Lage ist, sich an den Syndecan-1-Rezeptor zu binden, mit Ausnahme der Peptiden, die die Sequenz KKLRIKSKEK (SEQ ID 1) umfassen oder daraus bestehen.

**Claims**

1.  A synthetic peptide comprising or consisting of the sequence $Kaa_2aa_3Raa_5aa_6aa_7aa_8aa_9aa_{10}$, wherein, at physiological pH:

    - $aa_2$, $aa_6$, $aa_8$, $aa_{10}$ are positively charged residues ; at most one of them may be substituted by a neutrally charged residue ;
    - $aa_3$ is a neutrally charged residue,
    - $aa_5$ is a neutrally charged residue,
    - $aa_7$ is a neutrally charged residue with the exception of alanine, and
    - $aa_9$ is a negatively or neutrally charged residue

    the sequence being capable of binding to the syndecan-1 receptor,
    with the exception of the peptides comprising or consisting of the sequence KKLRIKSKEK (SEQ ID 1).

2.  The peptide according to claim 1, **characterized in that** it comprises or consists of a sequence selected from the group consisting of:

    - KALRIKSKEK (SEQ ID 2),
    - KKARIKSKEK (SEQ ID 3),
    - KKLRAKSKEK (SEQ ID 4),
    - KKLRIASKEK (SEQ ID 5),
    - KKLRIKSAEK (SEQ ID 6),
    - KKLRIKSKAK (SEQ ID 7),
    - KKLRIKSKEA (SEQ ID 8),
    - KKLRLRSKER (SEQ ID 9),
    - KKLRHKSKEK (SEQ ID 10),
    - KKLRTKSKEK (SEQ ID 11),
    - KKLRKKSKEK (SEQ ID 12),
    - KKLRSKSKEK (SEQ ID 13),
    - KKLRIKLKEK (SEQ ID 14),
    - KKLRIKQKEK (SEQ ID 15),
    - KKLRIQSKEK (SEQ ID 16),
    - KKLRIKSQEK (SEQ ID 17),
    - KKLRIKSKEQ (SEQ ID 18).

3.  A synthetic peptide

    • comprising at most 30 amino acids and comprising or consisting of the sequence KKLRIKSKEK (SEQ ID 1), or
    • comprising or consisting of the sequence $Kaa_2aa_3Raa_5aa_6aa_7aa_8aa_9aa_{10}$, wherein, at physiological pH:

      - $aa_2$, $aa_6$, $aa_8$, $aa_{10}$ are positively charged residues ; at most one of them may be substituted by a neutrally charged residue ;
      - $aa_3$ is a neutrally charged residue,
      - $aa_5$ is a neutrally charged residue,
      - $aa_7$ is a neutrally charged residue with the exception of alanine, and
      - $aa_9$ is a negatively or neutrally charged residue

    the sequence being capable of binding to the syndecan-1 receptor,
    with the exception of the peptide comprising or consisting of the sequence KKLRIKSKEK (SEQ ID 1)
    for use as a medicinal product.

4.  A peptide for its use according to claim 3, **characterized in that** it contains or consists of a sequence selected from the group consisting of:

- KALRIKSKEK (SEQ ID 2),
- KKARIKSKEK (SEQ ID 3),
- KKLRAKSKEK (SEQ ID 4),
- KKLRIASKEK (SEQ ID 5),
- KKLRIKSAEK (SEQ ID 6),
- KKLRIKSKAK (SEQ ID 7),
- KKLRIKSKEA (SEQ ID 8),
- KKLRLRSKER (SEQ ID 9),
- KKLRHKSKEK (SEQ ID 10),
- KKLRTKSKEK (SEQ ID 11),
- KKLRKKSKEK (SEQ ID 12),
- KKLRSKSKEK (SEQ ID 13),
- KKLRIKLKEK (SEQ ID 14),
- KKLRIKQKEK (SEQ ID 15),
- KKLRIQSKEK (SEQ ID 16),
- KKLRIKSQEK (SEQ ID 17),
- KKLRIKSKEQ (SEQ ID 18).

5. A peptide for its use according to any of claims 3 to 4, **characterized in that** it is used to promote the healing of the skin.

6. A peptide for its use according to any of claims 3 to 4, **characterized in that** it is used to promote tissue regeneration.

7. A pharmaceutical or cosmetic composition comprising a synthetic peptide, said peptide:

• comprising at most 30 amino acids and comprising or consisting of the sequence KKLRIKSKEK (SEQ ID 1), or
• comprising or consisting of the sequence $Kaa_2aa_3Raa_5aa_6aa_7aa_8aa_9aa_{10}$, wherein, at physiological pH:

- $aa_2$, $aa_6$, $aa_8$, $aa_{10}$ are positively charged residues ; at most one of them may be substituted by a neutrally charged residue ;
- $aa_3$ is a neutrally charged residue,
- $aa_5$ is a neutrally charged residue,
- $aa_7$ is a neutrally charged residue with the exception of alanine, and
- $aa_9$ is a negatively or neutrally charged residue

the sequence being capable of binding to the syndecan-1 receptor,
with the exception of the peptide comprising or consisting of the sequence KKLRIKSKEK (SEQ ID 1).

8. The composition according to claim 7, **characterized in that** it is presented in the form of a cream, a hydrogel, a solution, an injectable formulation or a spray which may include autologous keratinocytes or mesenchymal cells.

9. The composition according to claim 7, **characterized in that** it is presented in the form of a support onto which is grafted or deposited, or within which is incorporated, the synthetic peptide, the support being in the form of a film or a matrix consisting of comprising:

- a biological material selected from the group consisting of collagen, gelatine, polysaccharide, hyaluronic acid, cellulose, carboxymethylcellulose, pectin, chitosan, human or animal acellular dermis, or
- a synthetic material selected from the group consisting of silicone, polyurethane, PLLA, or
- a textile dressing material selected from the group including consisting of cotton, polyester and polyamide.

10. Use of the composition according to claim 7 as a culture medium for *in vitro* culturing of epithelial or mesenchymal cells.

11. A support for the culture of epithelial cells comprising a support being in the form of a polystyrene culture dish, a film or a porous matrix made of biopolymer, a textile membrane or gel collagen, onto which is grafted or adsorbed a synthetic peptide , said peptide :

• comprising at most 30 amino acids and comprising or consisting of the sequence KKLRIKSKEK (SEQ ID 1), or
• comprising or consisting of the sequence $Kaa_2aa_3Raa_5aa_6aa_7aa_8aa_9aa_{10}$, wherein, at physiological pH:

- $aa_2$, $aa_6$, $aa_8$, $aa_{10}$ are positively charged residues ; at most one of them may be substituted by a neutrally charged residue ;
- $aa_3$ is a neutrally charged residue,
- $aa_5$ is a neutrally charged residue,
- $aa_7$ is a neutrally charged residue with the exception of alanine, and
- $aa_9$ is a negatively or neutrally charged residue

the sequence being capable of binding to the syndecan-1 receptor,

with the exception of the peptide comprising or consisting of the sequence KKLRIKSKEK (SEQ ID 1).

Figure 1

```
                     DTP  VASPRSVKVW  QDACSPLPKT  1360
QANHGALQFG  DIPTSHLLFK  LPQELLKPRS  QFAVDMQTTS  1400
SRGLVFHTGT  KNSFMALYLS  KGRLVFALGT  DGKKLRIKSK  1440
EKCNDGKWHT  VVFGHDGEKG  RLVVDGLRAR  EGSLPGNSTI  1480
SIRAPVYLGS  PPSGKPKSLP  TNSFVGCLKN  FQLDSKPLYT  1520
PSSSFGVSSC  LGGPLEKGIY  FSEEGGHVVL  AHSVLLGPEF  1560
KLVFSIRPRS  LTGILIHIGS  QPGKHLCVYL  EAGKVTASMD  1600
SGAGGTSTSV  TPKQSLCDGQ  WHSVAVTIKQ  HILHLELDTD  1640
SSYTAGQIPF  PPASTQEPLH  LGGAPANLTT  LRIPVWKSFF  1680
GCLRNIHVNH  IPVPVTEALE  VQGPVSLNGC  PDQ         1713
```

Figure 2

Figure 3

Figure 4

Figure 5

**A**

**B**

Figure 6

Figure 7

EP 2 406 275 B1

Figure 8

43

Figure 9

Figure 10

Figure 11

Figure 12

Figure 13

Figure 14

Figure 15

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 200066731 A
- WO 2006018551 A
- WO 2006025646 A
- JP 2006063033 B, Utani

- US 20050059602 A
- US 5654135 A
- FR 0901168


**Littérature non-brevet citée dans la description**

- **URUSHIBATA et al.** *Biochemistry,* vol. 2099 (48), 10522-10532
- **HASHIMOTO et al.** *Biomaterials,* 2004, vol. 25, 1407-1414
- **BOYCE ; HAM.** Cultivation, frozen storage, and clonal growth of normal human epidermal keratinocytes in serum free-media. *Tiss Cult. Meth.,* 1985, vol. 9, 83-93
- **AMANO S ; SCOTT IC ; TAKAHARA K ; KOCH M ; CHAMPLIAUD MF ; GERECKE DR ; KEENE DR ; HUDSON DL ; NISHIYAMA T ; LEE S.** *J. Biol. Chem.,* 2000, vol. 275, 22728-22735
- Integrin-mediated cellular interactions with laminins. **AUMAILLEY M ; GIMOND C ; ROUSSELLE P.** The laminins. Harwood Academic Publishers, 1996, 127-158
- **AUMAILLEY M ; ROUSSELLE P.** Laminins of the dermo-epidermal junction. *Matrix Biol.,* 1999, vol. 18, 19-28
- **BACHY S ; LETOURNEUR F ; ROUSSELLE P.** Syndecan-1 interaction with the LG4/5 domain in laminin-332 is essential for keratinocyte migration. *J Cell Physiol.,* 2008, vol. 214, 238-249
- **BAKER SE ; HOPKINSON SB ; FITCHMUN M ; ANDREASON GL ; FRASIER F ; PLOPPER G ; QUARANTA V ; JONES JC.** *J. Cell Sci.,* 1996, vol. 109, 2509-2520
- **BELIN V ; ROUSSELLE P.** Production of a recombinantly expressed laminin fragment by HEK293-EBNA cells cultured in suspension in a dialysis-based bioreactor. *Protein Expr. Purif.,* 2006, vol. 48, 43-48
- **BERNFIELD M ; GOTTE M ; PARK PW ; REIZES O ; FITZGERALD ML ; LINCECUM J ; ZAKO M.** Functions of cell surface heparan sulfate proteoglycans. *Ann. Rev. Biochem.,* 1999, vol. 68, 729-777
- Wound repair : overview and general considerations. **CLARK RAF.** The molecular and cellular biology of wound repair. Plenum Press, 1996, 3-50

- **DECLINE F ; ROUSSELLE P.** Keratinocyte migration requires alpha2beta1 integrin-mediated interaction with the laminin 5 gamma2 chain. *J. Cell Sci.,* 2001, vol. 114, 811-823
- **DECLINE F ; OKAMOTO O ; MALLEIN-GERIN F ; HELBERT B ; BERNAUD J ; RIGAL D ; ROUSSELLE P.** Keratinocyte motility induced by TGF-beta1 is accompanied by dramatic changes in cellular interactions with laminin 5. *Cell Motil. Cytoskeleton,* 2003, vol. 54, 64-80
- **ELENIUS K ; VAINIO S ; LAATO M ; SALMIVIRTA M ; THESLEFF I ; JALKANEN M.** Induced expression of syndecan in healing wounds. *J. Cell Biol.,* 1991, vol. 114, 585-595
- **FRANK DE ; CARTER WG.** Laminin 5 deposition regulates keratinocyte polarization and persistent migration. *J. Cell Sci.,* 2004, vol. 117, 1351-1363
- **GALLO R ; KIM C ; KOKENYESI R ; ADZICK NS ; BERNFIELD M.** Syndecans-1 and -4 are induced during wound repair of neonatal but not fetal skin. *J. Invest. Dermatol.,* 1996, vol. 107, 676-683
- **GHOHESTANI RF ; LI K ; ROUSSELLE P ; UITTO J.** Molecular organization of the cutaneous basement membrane zone. *Clin. Dermatol.,* 2001, vol. 19, 551-562
- **GOLDFINGER LE ; HOPKINSON SB ; DEHART GW ; COLLAWN S ; COUCHMAN JR ; JONES JC.** The alpha3 laminin subunit, alpha6beta4 and alpha3beta1 integrin coordinately regulate wound healing in cultured epithelial cells and in the skin. *J. Cell Sci.,* 1999, vol. 112, 2615-2629
- **GOLDFINGER LE ; STACK MS ; JONES JC.** Processing of laminin-5 and its functional consequences: role of plasmin and tissue-type plasminogen activator. *J. Cell Biol.,* 1998, vol. 141, 255-265
- **HYNES RO.** Integrins : versatility, modulation, and signaling in cell adhesion. *Cell,* 1992, vol. 69, 11-25

- **JAAKKOLA P ; KONTUSAARI S ; KAUPPI T ; MAATA A ; JALKANEN M.** Wound reepithelialization activates a growth factor-responsive enhancer in migrating keratinocytes. *FASEB J,* 1998, vol. 12, 959-969
- **LAMPE PD ; NGUYEN BP ; GIL S ; USUI M ; OLERUD J ; TAKADA Y ; CARTER WG.** Cellular interaction of integrin alpha3beta1 with laminin 5 promotes gap junctional communication. *J. Cell Biol.,* 1998, vol. 143, 1735-1747
- **LARJAVA H ; SALO T ; HAASPASALMI K ; KRAMER RH ; HEINO J.** Expression of integrins and basement membrane components by wound keratinocytes. *J. Clin. Invest.,* 1993, vol. 92, 1425-1435
- **MARINKOVICH MP ; LUNSTRUM GP ; BURGESON RE.** The anchoring filament protein kalinin is synthesized and secreted as a high molecular weight precursor. *J. Biol. Chem.,* 1992, vol. 267, 17900-17906
- **MASUDA R ; MOCHIZUKI M ; HOZUMI K ; TAKEDA A ; UCHINUMA E ; YAMASHINA S ; NOMIZU M ; KADOYA Y.** A novel cell-adhesive scaffold material for delivering keratinocytes reduces granulation tissue in dermal wounds. *Wound Repair Regen,* 2009, vol. 17, 127-135
- **NGUYEN BP ; RYAN MC ; GIL SG ; CARTER WG.** Deposition of laminin 5 in epidermal wounds regulates integrin signaling and adhesion. *Curr. Opin. Cell Biol.,* 2000, vol. 12, 554-562
- **NIESSEN CM ; HOGERVORST F ; JASPARS LH ; DE MELKER AA ; DELWEL GO ; HULSMAN EH ; KUIKMAN I ; SONNENBERG A.** The alpha 6 beta 4 integrin is a receptor for both laminin and kalinin. *Exp Cell Res,* 1994, vol. 211, 360-367
- **OKAMOTO O ; BACHY S ; ODENTHAL U ; BERNAUD J ; RIGAL D ; LORTAT-JACOB H ; SMYTH N ; ROUSSELLE P.** Normal human keratinocytes bind to the alpha3LG4/5 domain of unprocessed laminin-5 through the receptor syndecan-1. *J. Biol. Chem.,* 2003, vol. 278, 44168-44177
- **OKSALA O ; SALO T ; TAMMI R ; HAKKINEN L ; JALKANEN M ; INKI P ; LARJAVA H.** Expression of proteoglycans and hyaluronan during wound healing. *J. Histochem. Cytochem.,* 1995, vol. 43, 125-135
- **ROUSSELLE P ; AUMAILLEY M.** Kalinin is more efficient than laminin in promoting adhesion of primary keratinocytes and some other epithelial cells and has a different requirement for integrin receptors. *J. Cell Biol.,* 1994, vol. 125, 205-214
- **ROUSSELLE P ; KEENE DR ; CHAMPLIAUD MF ; VAN DER REST M ; BURGESON R.E.** Laminin 5 binds type VII collagen thru its NC1 domain. *J. Cell Biol.,* 1997, vol. 138, 719-728
- **ROUSSELLE P ; LUNSTRUM GP ; KEENE DR ; BURGESON RE.** Kalinin : an epithelium-specific basement membrane adhesion molecule that is a component of anchoring filaments. *J. Cell Biol.,* 1991, vol. 114, 567-576
- **RYAN MC ; LEE K ; MIYASHITA Y ; CARTER WG.** Targeted disruption of the LAMA3 gene in mice reveals abnormalities in survival and late stage differentiation of epithelial cells. *J. Cell Biol.,* 1999, vol. 145, 1309-1323
- **RYAN MC ; TIZARD R ; VANDEVANTER DR ; CARTER WG.** Cloning of the LamA3 gene encoding the alpha 3 chain of the adhesive ligand epiligrin. Expression in wound repair. *J. Biol. Chem.,* 1994, vol. 269, 22779-22787
- **SIGLE RO ; GIL SG ; BHATTACHARYA M ; RYAN MC ; YANG TM ; BROWN TA ; BOUTAUD A ; MIYASHITA Y ; OLERUD J ; CARTER WG.** Globular domains 4/5 of the laminin alpha3 chain mediate deposition of precursor laminin 5. *J. Cell Sci.,* 2004, vol. 117, 4481-4494
- **SONNENBERG A ; DE MELKER AA ; MARTINEZ DE VELASCO AM ; JANSSEN H ; CALAFAT J ; NIESSEN CM.** Formation of hemidesmosomes in cells of a transformed murine mammary tumor cell line and mechanisms involved in adherence of these cells to laminin and kalinin. *J. Cell Sci.,* 1993, vol. 106, 1083-1102
- **STEPP MA ; GIBSON HE ; GALA PH ; IGLESIA DD ; PAJOOHESH-GANJI A ; PAL-GHOSH S ; BROWN M ; AQUINO C ; SCHWARTZ AM ; GOLDBERGER O.** Defects in keratinocyte activation during wound healing in the syndecan-1-deficient mouse. *J. Cell Sci.,* 2002, vol. 115, 4517-4531
- **SULKA B ; LORTAT-JACOB H ; TERREUX R ; LETOURNEUR F ; ROUSSELLE P.** Tyrosine déphosphorylation of the syndecan-1 PDZ binding domain regulates syntenin-1 recruitment. *J. Biol. Chem.,* 19 Février 2009
- **TIMPL R ; TISI D ; TALTS JF ; ANDAC Z ; SASAKI T ; HOHENESTER E.** Structure and function of laminin LG modules. *Matrix Biol.,* 2000, vol. 19, 309-317
- **TISI D ; TALTS JF ; TIMPL R ; HOHENESTER E.** Structure of the C-terminal laminin G-like domain pair of the laminin alpha2 chain harbouring binding sites for alpha-dystroglycan and heparin. *Embo J.,* 2000, vol. 19, 1432-1440
- **TUNGGAL L ; RAVAUX J ; PESCH M ; SMOLA H ; KRIEG T ; GAILL F ; SASAKI T ; TIMPL R ; MAUCH, C ; AUMAILLEY M.** Defective laminin 5 processing in cylindroma cells. *Am. J. Pathol.,* 2002, vol. 160, 459-468

- **UTANI A ; NOMIZU M ; MATSUURA H ; KATO K ; KOBAYASHI T ; TAKEDA U ; AOTA S ; NIELSEN PK ; SHINKAI H.** A unique sequence of the laminin alpha 3 G domain binds to heparin and promotes cell adhesion through syndecan-2 and -4. *J. Biol. Chem.,* 2001, vol. 276, 28779-28788

- **VIVÈS R ; CRUBLET E ; ANDIEU JP ; GAGNON J ; ROUSSELLE P ; LORTAT-JACOB H.** A novel strategy for defining critical amino acid residues involved in protein/glycosaminoglycan interactions. *J. Biol. Chem.,* 2004, vol. 279, 54327-54333
- **WOODS, A ; COUCHMAN JR.** Integrin modulation by lateral association. *J. Biol. Chem.,* 2000, vol. 275, 24233-24236